# EUROPEAN PATENT SPECIFICATION

(11) **EP 1 471 074 B1**
(45) Date of publication and mention of the grant of the patent: **16.07.2008**
(21) Application number: 04103238.4
(22) Date of filing: 15.04.1999
(51) Int. Cl.: C07K 14/18, C07K 1/113

(54) **Methods for improving the conformation of proteins by means of reducing agents**
Verfahren zur Verbesserung der Proteinkonformation mit Hilfe von Reduktionsmitteln
Procédé pour l'amélioration de la conformation des protéines par utilisation des agents réduisant

(30) Priority: 17.04.1998 EP 98870087
(43) Date of publication of application: 27.10.2004
(62) Divisional of application: 99920678.2
(73) Proprietor: Innogenetics N.V., 9052 Gent (BE)
(72) Inventor: Maertens, Geert, 8310 Brugge (BE); Louwagie, Joost, 2070 Zwijndrecht (BE); Bosman, Alfons, 1745 Opwijk (BE); Sablon, Erwin, 1785 Merchtem (BE); Zrein, Maan, 59910 Bondues (FR)

(56) References cited:
- EP-A- 0 346 500
- EP-A- 0 361 830
- WO-A-93/00365
- WO-A-95/30686
- US-A- 4 616 078
- US-A- 5 585 258
- MASUDA K ET AL.: "Efficient production of the C-terminal domain of secretory leukoprotease inhibitor as a thrombin-cleavable fusion protein in Escherichia coli" PROTEIN ENGINEERING, vol. 9, no. 1, 1996, pages 101-106, XP001194868
- DIBELLA EE ET AL: "Expression and Folding of Recombinant Bovine Prethrombin-2 and Its Activation to Thrombin" 1995, JOURNAL OF BIOLOGICAL CHEMISTRY, THE AMERICAN SOCIETY OF BIOLOGICAL CHEMISTS, INC.,, US, PAGE(S) 163-169 , XP002149152 ISSN: 0021-9258 * page 165, left-hand column *
- FISCHER B ET AL: "ISOLATION, RENATURATION, AND FORMATION OF DISULFIDE BONDS OF EUKARYOTIC PROTEINS EXPRESSED IN ESCHERICHIA COLI AS INCLUSION BODIES" BIOTECHNOLOGY AND BIOENGINEERING. INCLUDING: SYMPOSIUM BIOTECHNOLOGY IN ENERGY PRODUCTION AND CONSERVATION, JOHN WILEY & SONS. NEW YORK, US, vol. 41, no. 1, 5 January 1993 (1993-01-05), pages 3-13, XP000605146 ISSN: 0006-3592

### SEQUENCE LISTING

<110> Innogenetics N.V.
<120> Improved Immunodiagnostic assays using reducing agents
<130> 85 EPDiv1
<140> PCT/EP99/02547
   <141> 1999-04-15
<150> EP 98870087.8
   <151> 1998-04-17
<160> 38
<170> PatentIn Ver. 2.1
<210> 1
   <211> 36
   <212> DNA
   <213> Hepatitis C virus
<400> 1
   gggccccacc atgggggttg cgaaggcggt ggactt 36
<210> 2
   <211> 35
   <212> DNA
   <213> Hepatitis C virus
<400> 2
   ctattagctg aaagtcgact gtctgggtga cagca 35
<210> 3
   <211> 279
   <212> PRT
   <213> Hepatitis C virus
<400> 3
   Met Gly Val Ala Lys Ala Val Asp Phe Ile Pro Val Glu Asn Leu Glu
    1               5                  10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Ala Gln Gly Phe Lys
       50                  55                  60
   Val Leu Val Leu Asn Pro Ser Val Ala Ala Thr Leu Gly Phe Gly Ala
   65                  70                  75                  80
   Tyr Met Ser Arg Ala His Gly Ile Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Met Ile
           115                 120                 125
   Cys Asp Glu Cys His Ser Thr Asp Ala Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Ala Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Glu Leu Ala Ala Lys Leu Thr Ala Leu
       210                 215                 220
   Gly Val Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Tyr Thr Gly Asp Phe Asp Ser Val Ile Asp Cys Asn Thr Cys Val
               260                 265                 270
   Thr Gln Thr Val Asp Phe Ser
           275
<210> 4
   <211> 279
   <212> PRT
   <213> Hepatitis C virus
<400> 4
   Met Gly Val Ala Lys Ala Val Asp Phe Ile Pro Val Glu Asn Leu Glu
    1               5                  10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Thr Val Pro Ala Val Tyr Ala Ala Gln Gly Phe Lys
       50                  55                  60
   Val Leu Val Leu Asn Pro Ser Val Ala Ala Thr Leu Gly Phe Gly Ala
   65                  70                  75                  80
   Tyr Met Ser Arg Ala His Gly Ile Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ala Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg Arg Leu Ile Phe Cys
           195                 200                 205
   His Ser Lys Lys Lys Cys Asp Glu Leu Ala Ala Lys Leu Thr Ala Leu
       210                 215                 220
   Gly Val Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Tyr Thr Gly Asp Phe Asp Ser Val Ile Asp Cys Asn Thr Cys Val
               260                 265                 270
   Thr Gln Thr Val Asp Phe Ser
           275
<210> 5
   <211> 279
   <212> PRT
   <213> Hepatitis C virus
<400> 5
   Met Gly Val Ala Lys Ala Val Asp Phe Ile Pro Val Glu Ser Leu Glu
   1               5                   10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Gly Gln Gly Tyr Lys
       50                  55                  60
   Val Leu Val Leu Asn Pro Ser Val Ala Ala Thr Leu Gly Leu Gly Ala
   65                  70                  75                  80
   Tyr Met Ser Lys Val His Gly Ile Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ala Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Cys Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Glu Leu Ala Ala Asn Leu Val Ala Leu
       210                 215                 220
   Gly Val Asn Pro Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Tyr Thr Gly Asp Phe Asp Ser Val Ile Asp Cys Asn Thr Cys Val
               260                 265                 270
   Thr Gln Thr Val Asp Phe Ser
           275
<210> 6
   <211> 279
   <212> PRT
   <213> Hepatitis C virus
<400> 6
   Met Gly Val Ala Lys Ala Val Asp Phe Ile Pro Val Glu Ser Leu Glu
   1               5                   10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Ile Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ala Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Glu Leu Ala Ala Lys Pro Val Ala Leu
       210                 215                 220
   Gly Val Asn Ala Val Ala Tyr Tyr Arg Gly Leu Asp Val Pro Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 7
   <211> 279
   <212> PRT
   <213> Hepatitis C virus
<400> 7
   Met Gly Val Ala Lys Ala Val Asp Phe Ile Pro Val Glu Ser Leu Glu
    1               5                  10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Ile Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ala Thr Ser Ile Leu Asp Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Glu Leu Ala Ala Lys Leu Val Ala Leu
       210                 215                 220
   Gly Val Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 8
   <211> 279
   <212> PRT
   <213> Hepatitis C virus
<400> 8
   Met Gly Val Ala Lys Ala Val Asp Phe Ile Pro Val Glu Ser Leu Glu
    1               5                  10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Met Leu Val Leu Asn Pro Ser Val Ala Ala Thr Leu Gly Phe Gly Ala
   65                  70                  75                  80
   Tyr Met Ser Lys Ala Tyr Gly Ile Asp Pro Asn Ile Arg Thr Gly Val
                   85                  90                  95
   Arg Thr Ile Thr Thr Gly Ser Pro Thr Thr Tyr Ser Thr Tyr Gly Lys
               100                 105                 110
   Phe Leu Ala Asp Gly Gly Arg Ser Gly Gly Ala Tyr Asp Ile Ile Ile
           115                 120                 125
   Cys Asp Glu Cys His Ser Thr Asp Ala Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Val Phe Cys
           195                 200                 205
   His Ser Lys Lys Lys Cys Asp Glu Leu Ala Ala Lys Leu Val Ala Leu
       210                 215                 220
   Gly Val Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Arg
           275
<210> 9
   <211> 279
   <212> PRT
   <213> Hepatitis C virus
<400> 9
   Met Gly Val Ala Lys Ala Val Asp Phe Ile Pro Val Glu Ser Met Glu
    1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Ile Asp Pro Gly Ile Arg Thr Gly Val
                   85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ser Thr Thr Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Asn Thr Gly Glu Ile Pro Phe Tyr Gly Lys
               180                 185                 190
   Ala Ile Pro Leu Glu Thr Ile Lys Gly Gly Arg His Leu Ile Phe Cys
           195                 200                 205
   His Ser Lys Lys Lys Cys Asp Glu Leu Ala Ala Lys Leu Ser Ser Leu
       210                 215                 220
   Gly Leu Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Ala
               260                 265                 270
   Thr Gln Thr Val Asp Phe Ser
           275
<210> 10
   <211> 279
   <212> PRT
   <213> Hepatitis C virus
<400> 10
   Met Gly Val Ala Lys Ala Val Asp Phe Ile Pro Val Glu Ser Met Glu
    1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Ser Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Ile Asp Pro Gly Ile Arg Thr Gly Val
                   85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ser Thr Thr Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Asn Thr Gly Glu Ile Pro Phe Tyr Gly Lys
               180                 185                 190
   Ala Ile Pro Leu Glu Thr Ile Lys Gly Gly Arg His Leu Ile Phe Cys
           195                 200                 205
   His Ser Lys Lys Lys Cys Asp Glu Leu Ala Ala Lys Leu Ser Ser Leu
       210                 215                 220
   Gly Leu Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Ala
               260                 265                 270
   Thr Gln Thr Val Asp Phe Ser
           275
<210> 11
   <211> 279
   <212> PRT
   <213> Hepatitis C virus
<400> 11
   Met Gly Val Ala Lys Ala Val Asp Phe Ile Pro Val Glu Ser Met Glu
    1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
    65                 70                  75                  80
   Tyr Met Ser Lys Ala His Gly Ile Asp Pro Gly Ile Arg Thr Gly Val
                   85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ser Thr Thr Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Asn Thr Gly Glu Ile Pro Phe Tyr Gly Lys
               180                 185                 190
   Ala Ile Pro Leu Glu Thr Ile Lys Gly Gly Arg His Leu Ile Phe Cys
           195                 200                 205
   His Ser Lys Lys Lys Cys Asp Glu Leu Ala Ala Lys Leu Ser Ser Leu
       210                 215                 220
   Gly Leu Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 12
   <211> 279
   <212> PRT
   <213> Hepatitis C virus
<400> 12
   Met Gly Val Ala Lys Ala Val Asp Phe Ile Pro Val Glu Ser Met Glu
    1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Ile Asp Pro Gly Ile Arg Thr Gly Val
                   85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ser Thr Thr Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Asn Thr Gly Glu Ile Pro Phe Tyr Gly Lys
               180                 185                 190
   Ala Ile Pro Leu Glu Thr Ile Lys Gly Gly Arg His Leu Ile Phe Cys
           195                 200                 205
   His Ser Lys Lys Lys Cys Asp Glu Leu Ala Ala Lys Leu Ser Ser Leu
       210                 215                 220
   Gly Leu Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 13
   <211> 279
   <212> PRT
   <213> Hepatitis C virus
<400> 13
   Met Gly Val Ala Lys Ala Val Asp Phe Ile Pro Val Glu Ser Met Glu
     1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
        50                  55                  60
   Val Leu Val Leu Asn Pro Ser Val Ala Ala Thr Leu Gly Phe Gly Ala
    65                  70                  75                  80
   Tyr Met Ser Lys Ala His Gly Ile Asp Pro Gly Ile Arg Thr Gly Val
                    85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Ser Thr Thr Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Asn Thr Gly Glu Ile Pro Phe Tyr Gly Lys
               180                 185                 190
   Ala Ile Pro Leu Glu Thr Ile Lys Gly Gly Arg His Leu Ile Phe Cys
           195                 200                 205
   His Ser Lys Lys Lys Cys Asp Glu Leu Ala Ala Lys Leu Ser Ser Leu
       210                 215                 220
   Gly Leu Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 14
   <211> 279
   <212> PRT
   <213> Hepatitis C virus
<400> 14
   Met Gly Val Ala Lys Ala Val Asp Phe Ile Pro Val Glu Asn Leu Glu
   1               5                   10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Val Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Val Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Lys Leu Ala Ala Lys Leu Val Ala Leu
       210                 215                 220
   Gly Ile Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Tyr Thr Gly Asp Phe Asp Ser Val Ile Asp Cys Asn Thr Cys Val
               260                 265                 270
   Thr Gln Thr Val Asp Ile Ser
           275
<210> 15
   <211> 279
   <212> PRT
   <213> Hepatitis C virus
<400> 15
   Met Gly Val Ala Lys Ala Val Asp Phe Ile Pro Val Glu Asn Leu Glu
   1               5                   10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Asp Ser
               35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Val Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asn Gly Gly Cys Ser Gly Gly Ala Tyr Asp Ile Ile Ile
           115                 120                 125
   Cys Asp Glu Cys His Ser Thr Asp Val Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Glu Leu Ala Ala Lys Leu Val Ala Leu
       210                 215                 220
   Gly Ile Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Tyr Thr Gly Asp Phe Asp Ser Val Ile Asp Cys Asn Thr Cys Val
               260                 265                 270
   Thr Gln Thr Val Asp Phe Ser
           275
<210> 16
   <211> 279
   <212> PRT
   <213> Hepatitis C virus
<400> 16
   Met Gly Val Ala Lys Ala Val Asp Phe Ile Pro Val Glu Asn Leu Glu
   1               5                   10                  15
   Thr Thr Met Arg Ser Pro Val Phe Thr Asp Asn Ser Ser Pro Pro Ala
               20                  25                  30
   Val Pro Gln Ser Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Val Asp Pro Asn Ile Arg Thr Gly Val
                   85                  90                  95
   Arg Thr Ile Thr Thr Gly Ser Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Thr Asp Val Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Thr Thr Gly Glu Ile Pro Phe Tyr Gly Lys
               180                 185                 190
   Ala Ile Pro Leu Glu Ala Ile Lys Gly Gly Arg His Leu Ile Phe Cys
           195                 200                 205
   His Ser Lys Lys Lys Cys Asp Glu Leu Ala Ala Lys Gln Val Ala Leu
       210                 215                 220
   Gly Ile Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Tyr Thr Gly Asp Phe Asp Ser Val Ile Asp Cys Asn Thr Cys Val
               260                 265                 270
   Thr Gln Thr Val Asp Phe Ser
           275
<210> 17
   <211> 279
   <212> PRT
   <213> Hepatitis C virus
<400> 17
   Met Gly Val Ala Lys Ala Val Asp Phe Val Pro Val Glu Ser Met Glu
    1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Val Asp Pro Asn Ile Arg Thr Gly Val
                   85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Ile Asp Ser Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Ser Ile Gly Glu Ile Pro Phe Tyr Gly Lys
               180                 185                 190
   Ala Ile Pro Ile Glu Val Ile Lys Gly Gly Arg His Leu Ile Phe Cys
           195                 200                 205
   His Ser Lys Lys Lys Cys Asp Glu Leu Ala Ala Lys Leu Ser Gly Val
       210                 215                 220
   Gly Ile Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 18
   <211> 279
   <212> PRT
   <213> Hepatitis C virus
<400> 18
   Met Gly Val Ala Lys Ala Val Asp Phe Val Pro Val Glu Ser Met Glu
    1               5                  10                  15
   Thr Thr Met Arg Ser Pro Val Phe Thr Asp Asn Ser Ser Pro Pro Ala
               20                  25                  30
   Val Pro Gln Thr Phe Gln Val Ala His Leu His Ala Pro Thr Gly Ser
           35                  40                  45
   Gly Lys Ser Thr Lys Val Pro Ala Ala Tyr Ala Ala Gln Gly Tyr Lys
       50                  55                  60
   Val Leu Val Leu Asn Pro Ser Val Ala Ala Thr Leu Gly Phe Gly Ala
   65                  70                  75                  80
   Tyr Met Ser Lys Ala His Gly Val Asp Pro Asn Ile Arg Thr Gly Val
                   85                  90                  95
   Arg Thr Ile Thr Thr Gly Ala Pro Ile Thr Tyr Ser Thr Tyr Gly Lys
               100                 105                 110
   Phe Leu Ala Asp Gly Gly Cys Ser Gly Gly Ala Tyr Asp Ile Ile Ile
           115                 120                 125
   Cys Asp Glu Cys His Ser Ile Asp Ser Thr Ser Ile Leu Gly Ile Gly
       130                 135                 140
   Thr Val Leu Asp Gln Ala Glu Thr Ala Gly Ala Arg Leu Val Val Leu
   145                 150                 155                 160
   Ala Thr Ala Thr Pro Pro Gly Ser Val Thr Val Pro His Pro Asn Ile
                   165                 170                 175
   Glu Glu Val Ala Leu Ser Ser Thr Gly Glu Ile Pro Phe Tyr Gly Lys
               180                 185                 190
   Ala Ile Pro Ile Glu Val Ile Lys Gly Gly Arg His Leu Ile Phe Cys
           195                 200                 205
   His Ser Lys Lys Lys Cys Asp Glu Leu Ala Ala Lys Leu Ser Gly Phe
       210                 215                 220
   Gly Ile Asn Ala Val Ala Tyr Tyr Arg Gly Leu Asp Val Ser Val Ile
   225                 230                 235                 240
   Pro Thr Ser Gly Asp Val Val Val Val Ala Thr Asp Ala Leu Met Thr
                   245                 250                 255
   Gly Phe Thr Gly Asp Phe Asp Ser Val Ile Asp Cys Asn Thr Cys Val
               260                 265                 270
   Thr Gln Thr Val Asp Phe Ser
           275
<210> 19
   <211> 957
   <212> DNA
   <213> Hepatitis C virus
<400> 19
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   ggggttgcga aggcggtgga ctttgtaccc gtagagtcta tggaaaccac catgcggtcc 180
   ccggtcttta cggataactc atctcctccg gccgtaccgc agacattcca agtggcccat 240
   ctacacgccc ccactggtag tggcaagagc actaaggtgc cggctgcata tgcagcccaa 300
   gggtacaagg tacttgtcct gaacccatcc gttgccgcca ccttaggatt cggggcgtat 360
   atgtctaaag cacatggtgt cgaccctaac attagaactg gggtaaggac catcaccacg 420
   ggcgccccca ttacgtactc cacctacggc aagtttcttg ccgacggtgg ttgctctggg 480
   ggcgcttacg acatcataat atgtgatgag tgccactcga ttgactcaac ctccatcttg 540
   ggcatcggca ccgtcctgga tcaggcggag acggctggag cgcggcttgt cgtgctcgcc 600
   actgctacac ctccggggtc ggtcaccgtg ccacatccca acatcgagga ggtggctctg 660
   tccagcactg gagagatccc cttttatggc aaagccatcc ccatcgaggt catcaaaggg 720
   gggaggcacc tcattttctg ccattccaag aagaaatgtg acgagctcgc cgcaaagcta 780
   tcgggcttcg gaatcaacgc tgtagcgtat taccgaggcc ttgatgtgtc cgtcataccg 840
   actagcggag acgtcgttgt tgtggcaaca gacgctctaa tgacgggctt taccggcgac 900
   tttgactcag tgatcgactg taacacatgc gtcacccaga cagtcgactt cagctaa    957
<210> 20
   <211> 318
   <212> PRT
   <213> Hepatitis C virus
<400> 20
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Gly Val Ala Lys Ala Val Asp Phe
           35                  40                  45
   Val Pro Val Glu Ser Met Glu Thr Thr Met Arg Ser Pro Val Phe Thr
       50                  55                  60
   Asp Asn Ser Ser Pro Pro Ala Val Pro Gln Thr Phe Gln Val Ala His
   65                  70                  75                  80
   Leu His Ala Pro Thr Gly Ser Gly Lys Ser Thr Lys Val Pro Ala Ala
                   85                  90                  95
   Tyr Ala Ala Gln Gly Tyr Lys Val Leu Val Leu Asn Pro Ser Val Ala
               100                 105                 110
   Ala Thr Leu Gly Phe Gly Ala Tyr Met Ser Lys Ala His Gly Val Asp
           115                 120                 125
   Pro Asn Ile Arg Thr Gly Val Arg Thr Ile Thr Thr Gly Ala Pro Ile
       130                 135                 140
   Thr Tyr Ser Thr Tyr Gly Lys Phe Leu Ala Asp Gly Gly Cys Ser Gly
   145                 150                 155                 160
   Gly Ala Tyr Asp Ile Ile Ile Cys Asp Glu Cys His Ser Ile Asp Ser
                   165                 170                 175
   Thr Ser Ile Leu Gly Ile Gly Thr Val Leu Asp Gln Ala Glu Thr Ala
               180                 185                 190
   Gly Ala Arg Leu Val Val Leu Ala Thr Ala Thr Pro Pro Gly Ser Val
           195                 200                 205
   Thr Val Pro His Pro Asn Ile Glu Glu Val Ala Leu Ser Ser Thr Gly
           210                 215                 220
   Glu Ile Pro Phe Tyr Gly Lys Ala Ile Pro Ile Glu Val Ile Lys Gly
   225                 230                 235                 240
   Gly Arg His Leu Ile Phe Cys His Ser Lys Lys Lys Cys Asp Glu Leu
                   245                 250                 255
   Ala Ala Lys Leu Ser Gly Phe Gly Ile Asn Ala Val Ala Tyr Tyr Arg
               260                 265                 270
   Gly Leu Asp Val Ser Val Ile Pro Thr Ser Gly Asp Val Val Val Val
           275                 280                 285
   Ala Thr Asp Ala Leu Met Thr Gly Phe Thr Gly Asp Phe Asp Ser Val
       290                 295                 300
   Ile Asp Cys Asn Thr Cys Val Thr Gln Thr Val Asp Phe Ser
   305                 310                 315
<210> 21
   <211> 957
   <212> DNA
   <213> Hepatitis C virus
<400> 21
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   ggggttgcga aggcggtgga ctttatcccc gtggagagcc tagaaacaac catgaggtcc 180
   ccggtgttca cagacaactc ctccccgcca gcagtgcccc agagcttcca ggtggcccac 240
   ctgcatgctc ccaccggcag cggtaagagc accaaggtcc cggccgcata tgcggctcag 300
   ggctacaaag tgctggtgct caacccctcc gttgctgcaa cattgggctt tggtgcttac 360
   atgtccaagg cccatgggat tgatcctaac atcaggactg gggtaaggac aattactact 420
   ggcagcccca tcacgtactc cacctacggc aagttccttg ccgacggcgg gtgctcgggg 480
   ggtgcttatg acataataat ttgtgacgag tgccactcca cagatgcaac atctattttg 540
   ggcatcggca ctgtccttga ccaagcagag actgcggggg cgagactggt tgtgcttgcc 600
   accgctaccc ctccgggctc cgtcactgtg ccccatccta atatcgagga ggttgctctg 660
   tccaccaccg gagagatccc cttttacggc aaggctatcc cccttgaggc aatcaaaggg 720
   gggagacatc tcatcttctg ccactcaaag aagaagtgcg acgaactcgc cgccaaaccg 780
   gtcgcgttgg gtgtcaatgc cgtggcttac taccgcggcc ttgacgtgcc cgtcatcccg 840
   accagtggcg atgttgtcgt cgtggcaact gatgctctca tgaccggttt taccggtgac 900
   ttcgactcgg tgatagactg taatacgtgt gtcacccaga cagtcgactt cagctaa    957
<210> 22
   <211> 318
   <212> PRT
   <213> Hepatitis C virus
<400> 22
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Gly Val Ala Lys Ala Val Asp Phe
           35                  40                  45
   Ile Pro Val Glu Ser Leu Glu Thr Thr Met Arg Ser Pro Val Phe Thr
       50                  55                      60
   Asp Asn Ser Ser Pro Pro Ala Val Pro Gln Ser Phe Gln Val Ala His
   65                  70                      75                  80
   Leu His Ala Pro Thr Gly Ser Gly Lys Ser Thr Lys Val Pro Ala Ala
                   85                  90                      95
   Tyr Ala Ala Gln Gly Tyr Lys Val Leu Val Leu Asn Pro Ser Val Ala
               100                 105                     110
   Ala Thr Leu Gly Phe Gly Ala Tyr Met Ser Lys Ala His Gly Ile Asp
           115                 120                     125
   Pro Asn Ile Arg Thr Gly Val Arg Thr Ile Thr Thr Gly Ser Pro Ile
       130                 135                     140
   Thr Tyr Ser Thr Tyr Gly Lys Phe Leu Ala Asp Gly Gly Cys Ser Gly
   145                 150                     155                 160
   Gly Ala Tyr Asp Ile Ile Ile Cys Asp Glu Cys His Ser Thr Asp Ala
                   165                 170                     175
   Thr Ser Ile Leu Gly Ile Gly Thr Val Leu Asp Gln Ala Glu Thr Ala
               180                 185                     190
   Gly Ala Arg Leu Val Val Leu Ala Thr Ala Thr Pro Pro Gly Ser Val
           195                 200                     205
   Thr Val Pro His Pro Asn Ile Glu Glu Val Ala Leu Ser Thr Thr Gly
       210                 215                     220
   Glu Ile Pro Phe Tyr Gly Lys Ala Ile Pro Leu Glu Ala Ile Lys Gly
   225                 230                     235                 240
   Gly Arg His Leu Ile Phe Cys His Ser Lys Lys Lys Cys Asp Glu Leu
                   245                 250                     255
   Ala Ala Lys Pro Val Ala Leu Gly Val Asn Ala Val Ala Tyr Tyr Arg
               260                 265                     270
   Gly Leu Asp Val Pro Val Ile Pro Thr Ser Gly Asp Val Val Val Val
           275                 280                     285
   Ala Thr Asp Ala Leu Met Thr Gly Phe Thr Gly Asp Phe Asp Ser Val
       290                 295                     300
   Ile Asp Cys Asn Thr Cys Val Thr Gln Thr Val Asp Phe Ser
   305                 310                     315
<210> 23
   <211> 981
   <212> DNA
   <213> Hepatitis C virus
<400> 23
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   gccgcgggat tgggcccccc cataggtgta gcaaaagccc tacagttcat accagtggaa 180
   acccttagta cgcaggctag gtctccatct ttctctgaca attcaactcc tcctgctgtc 240
   ccacagagct atcaagtagg gtatcttcat gccccgaccg gcagcggtaa gagcacaaag 300
   gtcccggccg cttatgtagc acaaggatat aatgttctcg tgctgaatcc atcggtggcg 360
   gccacactag gcttcggctc tttcatgtcg cgtgcctatg ggatcgaccc caacatccgc 420
   actgggaacc gcaccgtcac aactggtgct aaactgacct attccaccta cggtaagttt 480
   ctcgcggacg ggggttgctc cgggggggca tatgatgtaa ttatctgtga tgaatgtcat 540
   gcccaagacg ccactagcat attgggcata ggcacggtct tagatcaggc cgagacggct 600
   ggggtgaggc tgacggtttt agcgacagca actcccccag gcagcatcac tgtgccacat 660
   tctaacatcg aggaagtggc cctgggctct gaaggtgaga tccctttcta cggtaaggct 720
   ataccgatag ccctgctcaa gggggggaga cacctcgtct tttgccattc caagaaaaaa 780
   tgtgatgagc tagcatccaa actcagaggt atggggctca acgctgtggc gtactatagg 840
   ggtctcgatg tttccgtcat accaacaaca ggagacgtcg tggtctgcgc tactgacgcc 900
   ctcatgactg gattcactgg agacttcgat tctgtcatag actgcaacgt ggctgttgaa 960
   cagtacgttg acttcagcta a                                           981
<210> 24
   <211> 326
   <212> PRT
   <213> Hepatitis C virus
<400> 24
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Ala Ala Gly Leu Gly Pro Pro Ile
           35                  40                  45
   Gly Val Ala Lys Ala Leu Gln Phe Ile Pro Val Glu Thr Leu Ser Thr
       50                  55                  60
   Gln Ala Arg Ser Pro Ser Phe Ser Asp Asn Ser Thr Pro Pro Ala Val
   65                  70                  75                  80
   Pro Gln Ser Tyr Gln Val Gly Tyr Leu His Ala Pro Thr Gly Ser Gly
                   85                  90                  95
   Lys Ser Thr Lys Val Pro Ala Ala Tyr Val Ala Gln Gly Tyr Asn Val
               100                 105                 110
   Leu Val Leu Asn Pro Ser Val Ala Ala Thr Leu Gly Phe Gly Ser Phe
           115                 120                 125
   Met Ser Arg Ala Tyr Gly Ile Asp Pro Asn Ile Arg Thr Gly Asn Arg
       130                 135                 140
   Thr Val Thr Thr Gly Ala Lys Leu Thr Tyr Ser Thr Tyr Gly Lys Phe
   145                 150                 155                 160
   Leu Ala Asp Gly Gly Cys Ser Gly Gly Ala Tyr Asp Val Ile Ile Cys
                   165                 170                 175
   Asp Glu Cys His Ala Gln Asp Ala Thr Ser Ile Leu Gly Ile Gly Thr
               180                 185                 190
   Val Leu Asp Gln Ala Glu Thr Ala Gly Val Arg Leu Thr Val Leu Ala
           195                 200                 205
   Thr Ala Thr Pro Pro Gly Ser Ile Thr Val Pro His Ser Asn Ile Glu
       210                 215                 220
   Glu Val Ala Leu Gly Ser Glu Gly Glu Ile Pro Phe Tyr Gly Lys Ala
   225                 230                 235                 240
   Ile Pro Ile Ala Leu Leu Lys Gly Gly Arg His Leu Val Phe Cys His
                   245                 250                 255
   Ser Lys Lys Lys Cys Asp Glu Leu Ala Ser Lys Leu Arg Gly Met Gly
               260                 265                 270
   Leu Asn Ala Val Ala Tyr Tyr Arg Gly Leu Asp Val Ser Val Ile Pro
           275                 280                 285
   Thr Thr Gly Asp Val Val Val Cys Ala Thr Asp Ala Leu Met Thr Gly
       290                 295                 300
   Phe Thr Gly Asp Phe Asp Ser Val Ile Asp Cys Asn Val Ala Val Glu
   305                 310                 315                 320
   Gln Tyr Val Asp Phe Ser
                   325
<210> 25
   <211> 981
   <212> DNA
   <213> Hepatitis C virus
<400> 25
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   gccgcgggat tgggccccac cataggtgta gcaaaagccc tacagttcat accagtggaa 180
   acccttagca cacaggctag gtctccatct ttctctgaca attcaactcc tcctgctgtt 240
   ccacagagct atcaagtagg gtaccttcat gccccgaccg gcagcggtaa gagcacaaag 300
   gtcccggccg cttatgtagc acaaggatat actgttctcg tgctgaatcc atcggtggcg 360
   gccacactag gcttcggctc tttcatgtcg cgtgcctatg ggatcgaccc caacatccgc 420
   actgggaacc gcaccgttac aactggtgct aaactgacct attccaccta cggtaagttt 480
   cttgcggatg ggggttgctc cgggggggca tatgatgtga ttatctgtga tgagtgtcat 540
   gcccaagacg ctactagcat attgggtata ggcacggtct tagatcaggc cgagacggct 600
   ggggtgaggc tgacggtttt agcgacagcg acccccccag gcagcatcac tgtgccacat 660
   tctaacatcg aagaagtggc cctgggctct gagggtgaga tccccttcta cggcaaggct 720
   ataccgatat ccctgctcaa gggggggagg caccttatct tttgccattc caaaaaaaag 780
   tgtgataaga tagcgtccaa actcagaggc atggggctca acgctgtagc gtactataga 840
   ggtctcgatg tgtccgtcat accaacaaca ggagacgtcg tagtttgcgc tactgacgcc 900
   ctcatgactg gatacaccgg ggacttcgat tctgtcatag actgcaacgt ggctgttgaa 960
   cagtacgttg acttcagcta a                                          981
<210> 26
   <211> 326
   <212> PRT
   <213> Hepatitis C virus
<400> 26
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Ala Ala Gly Leu Gly Pro Thr Ile
           35                  40                  45
   Gly Val Ala Lys Ala Leu Gln Phe Ile Pro Val Glu Thr Leu Ser Thr
       50                  55                  60
   Gln Ala Arg Ser Pro Ser Phe Ser Asp Asn Ser Thr Pro Pro Ala Val
   65                  70                  75                  80
   Pro Gln Ser Tyr Gln Val Gly Tyr Leu His Ala Pro Thr Gly Ser Gly
                   85                  90                  95
   Lys Ser Thr Lys Val Pro Ala Ala Tyr Val Ala Gln Gly Tyr Thr Val
               100                 105                 110
   Leu Val Leu Asn Pro Ser Val Ala Ala Thr Leu Gly Phe Gly Ser Phe
           115                 120                 125
   Met Ser Arg Ala Tyr Gly Ile Asp Pro Asn Ile Arg Thr Gly Asn Arg
       130                 135                 140
   Thr Val Thr Thr Gly Ala Lys Leu Thr Tyr Ser Thr Tyr Gly Lys Phe
   145                 150                 155                 160
   Leu Ala Asp Gly Gly Cys Ser Gly Gly Ala Tyr Asp Val Ile Ile Cys
                   165                 170                 175
   Asp Glu Cys His Ala Gln Asp Ala Thr Ser Ile Leu Gly Ile Gly Thr
               180                 185                 190
   Val Leu Asp Gln Ala Glu Thr Ala Gly Val Arg Leu Thr Val Leu Ala
           195                 200                 205
   Thr Ala Thr Pro Pro Gly Ser Ile Thr Val Pro His Ser Asn Ile Glu
       210                 215                 220
   Glu Val Ala Leu Gly Ser Glu Gly Glu Ile Pro Phe Tyr Gly Lys Ala
   225                 230                 235                 240
   Ile Pro Ile Ser Leu Leu Lys Gly Gly Arg His Leu Ile Phe Cys His
                   245                 250                 255
   Ser Lys Lys Lys Cys Asp Lys Ile Ala Ser Lys Leu Arg Gly Met Gly
               260                 265                 270
   Leu Asn Ala Val Ala Tyr Tyr Arg Gly Leu Asp Val Ser Val Ile Pro
           275                 280                 285
   Thr Thr Gly Asp Val Val Val Cys Ala Thr Asp Ala Leu Met Thr Gly
       290                 295                 300
   Tyr Thr Gly Asp Phe Asp Ser Val Ile Asp Cys Asn Val Ala Val Glu
   305                 310                 315                 320
   Gln Tyr Val Asp Phe Ser
                   325
<210> 27
   <211> 957
   <212> DNA
   <213> Hepatitis C virus
<400> 27
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   ggcgtggcca agtccataga cttcatcccc gttgagacac tcgacatcgt tacgcggtcc 180
   cccaccttta gtgacaacag cacgccaccg gctgtgcccc agacctatca ggtcgggtac 240
   ttgcatgccc caaccggcag cggaaagagc accaaagtcc ccgtcgcata cgccgcccag 300
   gggtataaag tgttagtgct caatccctcg gtggctgcta ccctggggtt tggagcgtac 360
   ctgtccaagg cacacggcat caatcccaac attaggactg gagtcaggac tgtgacgact 420
   ggcgaagcca tcacgtactc cacgtatggc aaattcctcg ccgatggggg ctgcgcaggt 480
   ggcgcctatg acatcatcat atgcgatgaa tgccacgccg tggatgccac taccattctc 540
   ggcatcggaa cagtccttga ccaagcagag acagccgggg tcaggctaac tgtgctggct 600
   acggccacgc cccccgggtc agtgacaacc ccccatccca acatagagga ggtagccctc 660
   gggcaggagg gtgagacccc cttctatggg agggcgatcc ccctgtctta catcaaggga 720
   gggagacact tgatcttctg ccactcaaag aaaaagtgtg acgagctcgc ggcggccctc 780
   cggggcatgg gcctgaacgc tgtggcgtac tacagagggc tcgacgtctc cgtaatacca 840
   gctcagggag atgtagtggt cgtcgccacc gacgccctca tgacggggtt cactggagac 900
   tttgactccg tgatcgactg caatgtagcg gtcactcaag ttgtagactt cagctaa    957
<210> 28
   <211> 318
   <212> PRT
   <213> Hepatitis C virus
<400> 28
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Gly Val Ala Lys Ser Ile Asp Phe
           35                  40                  45
   Ile Pro Val Glu Thr Leu Asp Ile Val Thr Arg Ser Pro Thr Phe Ser
       50                  55                  60
   Asp Asn Ser Thr Pro Pro Ala Val Pro Gln Thr Tyr Gln Val Gly Tyr
   65                  70                  75                  80
   Leu His Ala Pro Thr Gly Ser Gly Lys Ser Thr Lys Val Pro Val Ala
                   85                  90                  95
   Tyr Ala Ala Gln Gly Tyr Lys Val Leu Val Leu Asn Pro Ser Val Ala
               100                 105                 110
   Ala Thr Leu Gly Phe Gly Ala Tyr Leu Ser Lys Ala His Gly Ile Asn
           115                 120                 125
   Pro Asn Ile Arg Thr Gly Val Arg Thr Val Thr Thr Gly Glu Ala Ile
       130                 135                 140
   Thr Tyr Ser Thr Tyr Gly Lys Phe Leu Ala Asp Gly Gly Cys Ala Gly
   145                 150                 155                 160
   Gly Ala Tyr Asp Ile Ile Ile Cys Asp Glu Cys His Ala Val Asp Ala
                   165                 170                 175
   Thr Thr Ile Leu Gly Ile Gly Thr Val Leu Asp Gln Ala Glu Thr Ala
               180                 185                 190
   Gly Val Arg Leu Thr Val Leu Ala Thr Ala Thr Pro Pro Gly Ser Val
           195                 200                 205
   Thr Thr Pro His Pro Asn Ile Glu Glu Val Ala Leu Gly Gln Glu Gly
       210                 215                 220
   Glu Thr Pro Phe Tyr Gly Arg Ala Ile Pro Leu Ser Tyr Ile Lys Gly
   225                 230                 235                 240
   Gly Arg His Leu Ile Phe Cys His Ser Lys Lys Lys Cys Asp Glu Leu
                   245                 250                 255
   Ala Ala Ala Leu Arg Gly Met Gly Leu Asn Ala Val Ala Tyr Tyr Arg
               260                 265                 270
   Gly Leu Asp Val Ser Val Ile Pro Ala Gln Gly Asp Val Val Val Val
           275                 280                 285
   Ala Thr Asp Ala Leu Met Thr Gly Phe Thr Gly Asp Phe Asp Ser Val
       290                 295                 300
   Ile Asp Cys Asn Val Ala Val Thr Gln Val Val Asp Phe Ser
   305                 310                 315
<210> 29
   <211> 957
   <212> DNA
   <213> Hepatitis C virus
<400> 29
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   ggcgtagcca aatccattga cttcatccct gttgaatctc tcgatatcgc ctcacggtca 180
   cccagtttct ctgacaacag cacgccacca gctgtgcctc agtcctacca ggtgggctat 240
   ttgcacgcgc caacgggcag cgggaagagc accaaggtcc ctgtcgcata tgctagtcag 300
   gggtataaag tactcgtgct aaatccctct gtcgcggcca cgctcggctt cggggcctac 360
   atgtccaaag cccacgggat caaccccaac atcagaaccg gggtacggac tgtgaccacc 420
   ggggacccca tcacctactc cacttatggc aagtttctcg cagatggggg ctgctcagcc 480
   ggcgcctatg atgtcatcat atgcgatgaa tgccactcag tggacgctac taccatcctt 540
   ggcattggaa cagtcctcga ccaggccgag accgcgggtg ctaggttagt ggttttagcc 600
   acagccacgc ctcctggtac agtgacaact cctcatagca acatagagga ggtggctctt 660
   ggtcatgaag gcgagatccc tttctacggc aaggctattc ccctagcttt catcaagggg 720
   ggcagacacc taatcttttg ccattcaaag aagaagtgcg atgagctcgc ggcagccctt 780
   cggggcatgg gtgtcaacgc cgttgcttac tataggggtc tcgacgtctc tgttatacca 840
   actcaaggag acgtggtggt cgttgccacc gatgccctaa tgactggata caccggtgac 900
   tttgactctg ttattgactg caacgttgcg gtctctcaaa ttgtagactt cagctaa    957
<210> 30
   <211> 318
   <212> PRT
   <213> Hepatitis C virus
<400> 30
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Gly Val Ala Lys Ser Ile Asp Phe
           35                  40                  45
   Ile Pro Val Glu Ser Leu Asp Ile Ala Ser Arg Ser Pro Ser Phe Ser
       50                  55                  60
   Asp Asn Ser Thr Pro Pro Ala Val Pro Gln Ser Tyr Gln Val Gly Tyr
   65                  70                  75                  80
   Leu His Ala Pro Thr Gly Ser Gly Lys Ser Thr Lys Val Pro Val Ala
                   85                  90                  95
   Tyr Ala Ser Gln Gly Tyr Lys Val Leu Val Leu Asn Pro Ser Val Ala
               100                 105                 110
   Ala Thr Leu Gly Phe Gly Ala Tyr Met Ser Lys Ala His Gly Ile Asn
           115                 120                 125
   Pro Asn Ile Arg Thr Gly Val Arg Thr Val Thr Thr Gly Asp Pro Ile
       130                 135                 140
   Thr Tyr Ser Thr Tyr Gly Lys Phe Leu Ala Asp Gly Gly Cys Ser Ala
   145                 150                 155                 160
   Gly Ala Tyr Asp Val Ile Ile Cys Asp Glu Cys His Ser Val Asp Ala
                   165                 170                 175
   Thr Thr Ile Leu Gly Ile Gly Thr Val Leu Asp Gln Ala Glu Thr Ala
               180                 185                 190
   Gly Ala Arg Leu Val Val Leu Ala Thr Ala Thr Pro Pro Gly Thr Val
           195                 200                 205
   Thr Thr Pro His Ser Asn Ile Glu Glu Val Ala Leu Gly His Glu Gly
       210                 215                 220
   Glu Ile Pro Phe Tyr Gly Lys Ala Ile Pro Leu Ala Phe Ile Lys Gly
   225                 230                 235                 240
   Gly Arg His Leu Ile Phe Cys His Ser Lys Lys Lys Cys Asp Glu Leu
                   245                 250                 255
   Ala Ala Ala Leu Arg Gly Met Gly Val Asn Ala Val Ala Tyr Tyr Arg
               260                 265                 270
   Gly Leu Asp Val Ser Val Ile Pro Thr Gln Gly Asp Val Val Val Val
           275                 280                 285
   Ala Thr Asp Ala Leu Met Thr Gly Tyr Thr Gly Asp Phe Asp Ser Val
       290                 295                 300
   Ile Asp Cys Asn Val Ala Val Ser Gln Ile Val Asp Phe Ser
   305                 310                 315
<210> 31
   <211> 957
   <212> DNA
   <213> Hepatitis C virus
<400> 31
   atggtaagat caagtagtca aaattcgagt gacaagcctg tagcccacgt cgtagcaaac 60
   caccaagtgg aggagcaggg aattcaccat caccatcacc acgtggatcc cgggcccatg 120
   ggcgtagcca aatccattga cttcatcccc gttgagtctc tcgacatcgt gactaggtct 180
   ccaagcttca ctgacaacag tacacctcca gccgtgcctc agacctacca agtggggtat 240
   ctccacgcgc ccactggtag cgggaagagt accaaggtcc ctgcagcgta cgccgctcag 300
   gggtacaagg tgctggtact gaacccctcc gtggctgcca ctttgggatt tggggcctac 360
   atgtcaaaag cgcacggagt caatcccaat atcaggaccg gggttcgcac ggtgaacact 420
   ggggatccca tcacctactc cacgtatggc aaattcctcg cagatggagg ctgctctgga 480
   ggcgcctatg gcatcataat atgcgacgaa tgccattcga cggactccac gaccatcctc 540
   ggcatcggga ccgttctcga ccaagctgag acagctggag ttaggttggt ggtcttggcc 600
   acggcgaccc cacccggatc tgtaacaacc ccacacccca acatagagga ggtggccctc 660
   ggccacgagg gcgaaatccc cttctatggg aaggccatcc ctctctcaac catcaaggga 720
   ggacgacatc taatcttctg tcattcaaag aaaaagtgcg acgagctcgc ggtggccctc 780
   cgagcgatgg gccttaacgc ggtggcatac tacagagggc ttgacgtctc cgtgatacca 840
   acacaaggag acgtggtggt cgtcgccacc gacgccctca tgacaggata tactggagac 900
   ttcgactctg tgatcgactg caacatggcg gtctctcaaa ttgtagactt cagctaa    957
<210> 32
   <211> 318
   <212> PRT
   <213> Hepatitis C virus
<400> 32
   Met Val Arg Ser Ser Ser Gln Asn Ser Ser Asp Lys Pro Val Ala His
    1               5                  10                  15
   Val Val Ala Asn His Gln Val Glu Glu Gln Gly Ile His His His His
               20                  25                  30
   His His Val Asp Pro Gly Pro Met Gly Val Ala Lys Ser Ile Asp Phe
           35                  40                  45
   Ile Pro Val Glu Ser Leu Asp Ile Val Thr Arg Ser Pro Ser Phe Thr
       50                  55                  60
   Asp Asn Ser Thr Pro Pro Ala Val Pro Gln Thr Tyr Gln Val Gly Tyr
   65                  70                  75                  80
   Leu His Ala Pro Thr Gly Ser Gly Lys Ser Thr Lys Val Pro Ala Ala
                   85                  90                  95
   Tyr Ala Ala Gln Gly Tyr Lys Val Leu Val Leu Asn Pro Ser Val Ala
               100                 105                 110
   Ala Thr Leu Gly Phe Gly Ala Tyr Met Ser Lys Ala His Gly Val Asn
           115                 120                 125
   Pro Asn Ile Arg Thr Gly Val Arg Thr Val Asn Thr Gly Asp Pro Ile
       130                 135                 140
   Thr Tyr Ser Thr Tyr Gly Lys Phe Leu Ala Asp Gly Gly Cys Ser Gly
   145                 150                 155                 160
   Gly Ala Tyr Gly Ile Ile Ile Cys Asp Glu Cys His Ser Thr Asp Ser
                   165                 170                 175
   Thr Thr Ile Leu Gly Ile Gly Thr Val Leu Asp Gln Ala Glu Thr Ala
               180                 185                 190
   Gly Val Arg Leu Val Val Leu Ala Thr Ala Thr Pro Pro Gly Ser Val
           195                 200                 205
   Thr Thr Pro His Pro Asn Ile Glu Glu Val Ala Leu Gly His Glu Gly
       210                 215                 220
   Glu Ile Pro Phe Tyr Gly Lys Ala Ile Pro Leu Ser Thr Ile Lys Gly
   225                 230                 235                 240
   Gly Arg His Leu Ile Phe Cys His Ser Lys Lys Lys Cys Asp Glu Leu
                   245                 250                 255
   Ala Val Ala Leu Arg Ala Met Gly Leu Asn Ala Val Ala Tyr Tyr Arg
               260                 265                 270
   Gly Leu Asp Val Ser Val Ile Pro Thr Gln Gly Asp Val Val Val Val
           275                 280                 285
   Ala Thr Asp Ala Leu Met Thr Gly Tyr Thr Gly Asp Phe Asp Ser Val
       290                 295                 300
   Ile Asp Cys Asn Met Ala Val Ser Gln Ile Val Asp Phe Ser
   305                 310                 315
<210> 33
   <211> 36
   <212> DNA
   <213> Hepatitis C virus
<400> 33
   gggccccacc ataggtgtag caaaagccct acagtt 36
<210> 34
   <211> 33
   <212> DNA
   <213> Hepatitis C virus
<400> 34
   ctattagctg aagtcaacgt actgttcaac agc 33
<210> 35
   <211> 36
   <212> DNA
   <213> Hepatitis C virus
<400> 35
   gggccccacc atgggcgtgg ccaagtccat agactt 36
<210> 36
   <211> 33
   <212> DNA
   <213> Hepatitis C virus
<400> 36
   ctattagctg aagtctacaa cttgagtgac cgc 33
<210> 37
   <211> 36
   <212> DNA
   <213> Hepatitis C virus
<400> 37
   gggccccacc atgggcgtag ccaaatccat tgactt 36
<210> 38
   <211> 33
   <212> DNA
   <213> Hepatitis C virus
<400> 38
   ctattagctg aagtctacaa tttgagagac cgc 33

## Description

### FIELD OF THE INVENTION

The present invention relates to the field of diagnosis and treatment of HCV infection. More particularly, the present invention relates to HCV NS3 helicase and its uses. Also the present invention relates to improved immunodiagnostic assays.

### BACKGROUND OF THE INVENTION

Hepatitis C Viruses (HCV) constitute a genus within the Flaviviridae, with closest homology to the hepatitis G and GB viruses, and Pestiviruses. The positive-stranded RNA genome encodes at least 9 proteins. Core, E1, and E2 constitute the structural proteins. NS2, NS3, NS4A, NS4B, NS5A, and NS5B are non-structural (NS) proteins. HCV isolates display high levels of sequence heterogeneity allowing classification into at least 11 types and 90 subtypes (Maertens and Stuyver, 1997). HCV infection of the human liver is often clinically benign, with mild icterus in the acute phase. The disease may even go unnoticed in some cases of acute resolving hepatitis C. In the majority (>70%) of cases, however, HCV infection leads to chronic persistent or active infection, often with complications of liver cirrhosis and auto-immune disorders. Hepatocellular carcinoma may occur after about 20 to 35 years (Saito et al., 1990), sometimes even without the intermediate phase of cirrhosis. No prophylaxis is available today and treatment with interferon-alpha (IFN- ) only leads to long-term resolution in about 4 to 36% of treated cases, depending on the HCV genotype (Maertens and Stuyver, 1997).

Since productive culture methods for HCV are currently not available, and since only minute amounts of HCV antigens circulate in the infected patient, direct detection of HCV particles cannot be performed routinely, and indirect diagnosis is only possible using cumbersome amplification techniques for HCV RNA detection. Unlike with many other viral infections, HCV particles generally persist in the blood, liver, and lymphocytes despite the presence of cellular and humoral immune response to most of the HCV proteins. HCV antibodies can be conveniently detected by Elisa techniques which allow high throughput screening in blood banks and clinical laboratories. Supplementary antibody testing is required and is now mandatory in most countries. True HCV reactivity is thus discriminated from false reactivity, which may be caused by non-specific binding of serum or plasma immunoglobulines or anti-idiotypic components to the coating or blocking reagents, or to contaminants present in HCV antigen preparations, or even to fusion parts or non-specific regions of the recombinant antigens themselves (McFarlane et al., 1990). HCV RNA detection by PCR or branched DNA (bDNA) techniques have recently been introduced to monitor chronic HCV disease, especially during therapy. Surprisingly, HCV RNA detection is sometimes employed to confirm HCV Ab screening tests, despite the fact that only ~70-94% of repeatedly HCV Ab positive patient samples are positive by nested PCR (Marin et al., 1994). Of HCV Ab positive blood donors, who usually present with milder forms of the disease and low HCV RNA levels, confirmation by nested PCR is usually in the order of ~40% (Waumans et al., 1993; Stuyver et al., 1996). Strip-based assays therefore provide the only reliable alternative for HCV Ab confirmation. Even in the case of an indeterminate result in the confirmatory assay, serological follow up of the patient rather than HCV RNA detection is advisable (Di Bisceglie et al., 1998). Since native HCV antigens are not available in sufficient quantities, such confirmatory assays incorporate synthetic peptides and/or recombinant fragments of HCV proteins. One of the most critical issues in the confirmation of antibodies constitutes the reactivity of the NS3 protein (Zaaijer et al., 1994). NS3 antibodies often appear first in seroconversion series and the reactivity of the NS3 protein seems to be different in the different commercial assays available today .

Innogenetics introduced the concept of strip technology in which usually a combination of synthetic peptides and recombinant proteins are applied as discrete lines in an ordered and easily readable fashion. The INNO-LIA HIV Ab tests have proven to be superior to routinely used western blots (Pollet et al., 1990). The Line Immuno Assay allows multiparameter testing and thus enables incorporation of cutoff and other rating systems, sample addition control, as well as testing for false reactivity to non-HCV proteins used as carrier or fusion partner required for some antigens in the Elisa test. In principle, the test format allows to combine antigens of different different aetiological agents or phenotypically linked conditions into a single test. The INNO-LIA HCV Ab III is a 3rd generation Line Immuno Assay which incorporates HCV antigens derived from the Core region, the E2 hypervariable region (HVR), the NS3 helicase region, and the NS4A, NS4B, and NS5A regions. In the third generation assay, highly purified recombinant subtype 1b NS3 protein and E2 peptides enabled superior sensitivity while safeguarding the reliable specificity which is characteristic of peptide-based tests (Peeters et al., 1993). Perhaps one of the most important features of this assay is its unprecedented correlation with HCV RNA positivity (Claeys et al., 1992; De Beenhouwer et al., 1992).

The antigens are coated as 6 discrete lines on a nylon strip with plastic backing. In addition, four control lines are coated on each strip: anti-streptavidin, 3+ positive control (anti-human Ig), 1+ positive control (human IgG), and the cutoff line (human IgG). A diluted test sample is incubated in a trough together with the LIA III strip. If present in the sample, HCV antibodies will bind to the HCV antigen lines on the strip. Subsequently, an affinity-purified alkaline phosphatase labelled goat anti-human IgG (H+L) conjugate is added and reacts with specific HCV antigen/antibody complexes if previously formed. Incubation with enzyme substrate produces a chestnut-like color, the intensity of which is proportionate to the amount of HCV-specific antibody captured from the sample on any given line. Color development is stopped with sulphuric acid. If no HCV-specific antibodies are present, the conjugate only binds to the , 1+, and 3+ control lines. If the addition of sample is omitted, only the and 1+ control lines will be stained.

Expression of eukaryotic proteins containing cysteine, which may form disulfide bonds in the native active protein, often leads to the formation of inclusion bodies characterized by nonnative inter- and intramolecular disulfide bonds. The different steps and methods of isolation and renaturation of disulfide bonds of recombinant eukaryotic polypeptides expressed in *Escherichia coli* as inclusion bodies, have been reviewed by Fischer et al. (Biotechnology and bioengineering 41:3-13, 1993). Specific examples of such methods for recombinant expression of mammalian proteins in bacterial expression systems are described in EP 0 346 500, EP 0 361 830, US 4,616,078, WO 95/30686, DiBella et al. (Journal of Biological Chemistry 270:163-169, 1995) and Masuda et al. (Protein Engineering 9:101-106, 1996).

### DEFINITIONS

The following definitions serve to illustrate the different terms and expressions used in the present invention.

The term 'HCV NS3' protein refers to a polypeptide or an analogue thereof (e.g. mimotopes) comprising an amino acid sequence (and/or amino acid analogues) defining at least one HCV epitope of either HCV NS3 protease or helicase.

It should also be understood that the isolates (biological samples) used in the examples section of the present invention were not intended to limit the scope of the invention and that any HCV isolate belonging to type 1, 2, 3, 4, 5, 6, 7, 8, 9, 10, 11, 12 or any other new genotype of HCV is a suitable source of HCV sequence for the practice of the present invention.

The HCV antigens used in the present invention may be full-length viral proteins, substantially full-length versions thereof, or functional fragments thereof (e.g. fragments which are not missing sequence essential to the formation or retention of an epitope). Furthermore, the HCV antigens of the present invention can also include other sequences that do not block or prevent the formation of any conformational epitope of interest. The presence or absence of a conformational epitope can be readily determined though screening the antigen of interest with an antibody (polyclonal serum or monoclonal antibody) and comparing its reactivity to that of a denatured version of the antigen which retains only linear epitopes (if any). When in such screening polyclonal antibodies are used, it may be advantageous to adsorb the polyclonal serum first with the denatured antigen and see if it retains antibodies to the antigen of interest.

The term 'fusion polypeptide' intends a polypeptide in which the antigen(s), in particularly HCV antigen(s), are part of a single continuous chain of amino acids, which chain does not occur in nature. The HCV antigens may be connected directly to each other by peptide bonds or be separated by spacer amino acid sequences. The fusion polypeptides may also contain amino acid sequences exogenous to HCV.

The term 'purified' as applied to proteins herein refers to a composition wherein the desired protein comprises at least 35% of the total protein component in the composition. The desired protein preferably comprises at least 40%, more preferably at least about 50%, more preferably at least about 60%, still more preferably at least about 70%, even more preferably at least about 80%, even more preferably at least about 85%, even more preferably at least about 90%, and most preferably at least about 95% of the total protein component. The composition may contain other compounds such as carbohydrates, salts, lipids, solvents, and the like, without affecting the determination of the percentage purity as used herein. An 'isolated' HCV protein intends an HCV protein composition that is at least 35% pure.

The term 'essentially purified proteins' refers to proteins purified such that they can be used for in vitro diagnostic methods and as a therapeutic compound. These proteins are substantially free from cellular proteins or DNA, vector-derived proteins or DNA, or other HCV viral components. Usually these proteins are purified to homogeneity (at least 80% pure, preferably, 85%, more preferably, 90%, more preferably 95%, more preferably 97%, more preferably 98%, more preferably 99%, even more preferably 99.5%, and most preferably the contaminating proteins should be undetectable by conventional methods like SDS-PAGE and silver staining.

The term 'recombinantly expressed' used within the context of the present invention refers to the fact that the proteins of the present invention are produced by recombinant expression methods be it in prokaryotes, or lower or higher eukaryotes as discussed in detail below.

The term 'polypeptide' refers to a polymer of amino acids and does not refer to a specific length of the product; thus, peptides, oligopeptides, and proteins are included within the definition of polypeptide. This term also does not refer to or exclude post-expression modifications of the polypeptide, for example, glycosylations, acetylations, phosphorylations and the like. Included within the definition are, for example, polypeptides containing one or more analogues of an amino acid (including, for example, unnatural amino acids, PNA, etc.), polypeptides with substituted linkages, as well as other modifications known in the art, both naturally occurring and non-naturally occurring.

The term 'recombinant polynucleotide or nucleic acid' intends a polynucleotide or nucleic acid of genomic, cDNA, semisynthetic, or synthetic origin which, by virtue of its origin or manipulation: (1) is not associated with all or a portion of a polynucleotide with which it is associated in nature, (2) is linked to a polynucleotide other than that to which it is linked in nature, or (3) does not occur in nature.

The term 'recombinant host cells', 'host cells', 'cells', 'cell lines', 'cell cultures', and other such terms denoting microorganisms or higher eukaryotic cell lines cultured as unicellular entities refer to cells which can be or have been, used as recipients for a recombinant vector or other transfer polynucleotide, and include the progeny of the original cell which has been transfected. It is understood that the progeny of a single parental cell may not necessarily be completely identical in morphology or in genomic or total DNA complement as the original parent, due to natural, accidental, or deliberate mutation.

### AIMS OF THE INVENTION

It is an aim of the present invention to provide a method for purifying cysteine containing recombinant HCV NS3 proteins.

The aim of the present invention is considered to have been met by the embodiments as set out below.

### DETAILED DESCRIPTION OF THE INVENTION

As is demonstrated in the Examples section the present inventors have found that the presence of a reducing agent such as DTT, besides an antigen coated to a solid phase, renders a solid phase immunassay coupled antigen much more reactive with antibodies directed to said antigen. Also in solution, the antigen is rendered more reactive by reduction.

A reducing agent according to the present invention is any agent which achieves reduction of S-S disulfide bridges. Reduction of the 'S-S' disulfide bridges is a chemical reaction whereby the disulfides are reduced to thiol (-SH). The disulfide bridge breaking agents and methods disclosed in WO 96/04385 are hereby incorporated by reference in the present description. 'S-S' Reduction can be obtained by (1) enzymatic cascade pathways or by (2) reducing compounds. Enzymes like thioredoxin, glutaredoxin are known to be involved in the in vivo reduction of disulfides and have also been shown to be effective in reducing 'S-S' bridges in vitro. Disulfide bonds are rapidly cleaved by reduced thioredoxin at pH 7.0, with an apparent second order rate that is around 10⁴ times larger than the corresponding rate constant for the reaction with DTT. The reduction kinetic can be dramatically increased by preincubation the protein solution with 1 mM DTT or dihydrolipoamide (Holmgren, 1979).

Thiol compounds able to reduce protein disulfide bridges are for instance Dithiothreitol (DTT), Dithioerythritol (DTE), β-mercaptoethanol, thiocarbamates, bis(2-mercaptoethyl) sulfone and N,N'-bis(mercaptoacetyl)hydrazine, and sodium-dithionite.

Reducing agents without thiol groups like ascorbate or stannous chloride (SnCl₂), which have been shown to be very useful in the reduction of disulfide bridges in monoclonal antibodies (Thakur et al., 1991), may also be used for the reduction of NS3. Sodium borohydride treatment has been shown to be effective for the reduction of disulfide bridges in peptides (Gailit, 1993). Tris (2-carboxyethyl)phosphine (TCEP) is able to reduce disulfides at low pH (Burns et al., 1991). Selenol catalyses the reduction of disulfide to thiols when DTT or sodium borohydride is used as reductant. Selenocysteamine, a commercially available diselenide, was used as precursor of the catalyst (Singh and Kats, 1995).

Sulphonation and desulphonation is a reaction whereby -SO₃ groups are introduced or removed respectively from the protein.

Sulphonation is defined as a process where thiolgroups (SH) on proteins (R) and disulphide bonds are converted to S-Sulphonates, according to the following reactions:

RSH → RS-SO₃⁻ (1)

RS-SR + 2 -SO₃⁻+H₂O → 2 RS-SO₃⁻+ 2 OH⁻ (2)

The products of the reactions are S-Sulphoproteins which are usually stable at neutral pH. Reaction (1) can be obtained by incubation the protein solution with tetrathionate at pH>7 (Inglis and Liu, 1970). Reaction (2) proceeds to completion in the presence of copper ions (Cole, 1967). Chan (1968) has shown that treatment of protein with sodium sulfite and catalytic amounts of cysteine in the presence of oxygen gives sulpho-proteins.

Desulfonation can be obtained (1) by an excess of competitive -SH (thiol) groups, (2) by reducing agents or (3) by incubation in non-neutral pH conditions.

RS-SO₃- + R'SH → RSH + R'S-SO₃⁻

RS-SO₃⁻ + reducing agent → RSH

Competitive thiol groups may be obtained from low molecular weight compounds or from proteinacous -SH groups.

Examples of mono- or dithiol containing compounds are:
cysteine, cysteamine, reduced gluthation, N-acetyl cysteine, homocysteine, β-mercaptoethanol, thiocarbamates, bis(2-mercaptoethyl)sulphone (BMS) and N,N'-bis(mercaptoacetyl)hydrazine (BMH), 5,5'-dithiobis-(2-nitrobenzoic acid) (DTNB or Elman's reagent), Dithiotreitol (DTT) and Dithioerythrithiol (DTE).

The present invention further relates to a method for purifying a cysteine containing, recombinantly expressed HCV NS3 protein, comprising steps (a) and (c), and at least 1 of the following steps (b), (d) or (e):
(a) sulphonation of a lysate from recombinant host cells or lysis of recombinant host cells in the presence of guanidinium chloride (preferably 6 M Gu.HCl) and sulphonation of the cell lysate,
(b) treatment with a zwitterionic detergent, preferably after removal of the cell debris,
(c) purification of the sulphonated recombinant protein, or purification of the sulphonated recombinant protein with subsequent removal of the zwitterionic detergent, with said purification being preferably chromatography, more preferably a Ni-IMAC chromatography with said recombinant protein being a His-tagged recombinant protein,
(d) desulphonation of the sulphonated recombinant protein, preferably with a molar excess of a reducing agent such as DTT,
(e) storage in the presence of a molar excess of DTT, or immediate use in an assay. Empigen is a particularly preferred example of a zwitterionic detergent. Inclusion of such a zwitterionic detergent and DTT was found to improve the purification protocol for HCV NS3 helicase.

The present invention also relates to any method for producing and using said polyproteins of the invention. Methods for producing and using HCV polyproteins are disclosed in WO 96/13590. Said uses include not only diagnostic uses but also therapeutic and prophylactic uses. The NS3 proteins of the invention are also particularly suited to be incoporated in vaccine compositions. Said vaccine composition may contain, besides the active ingredient, any type of adjuvant known in the art. The NS3 proteins of the present invention may also be used in any application where it is applicable to use an NS3 helicase, such as for drug screening purposes.

### FIGURE LEGENDS

**Figure 1****.** Amino acid sequence of HCV NS3 clones isolated from HCV subtype 1a and 1b infected sera.
**Figure 2-1**. DNA coding sequence of the mTHFH6NS3 clone 19b fusion protein. Sequence depicted in bold is non-NS3 sequence. This sequence encodes the mTNF fusionpartner, the hexahistidine tag and part of the multilinker.
**Figure 2-2****.** Amino Acid sequence of the mTNFH6NS3 clone 19b fusionprotein. Sequence depicted in bold is non-NS3 sequence. This sequence contains the mTNF fusionpartner, the hexahistidine tag and part of the multilinker.
**Figure 3-1**. DNA coding sequence of the mTNFH6NS3 clone B9 fusionprotein. Sequence depicted in bold is non-NS3 sequence. This sequence encodes the mTNF fusionpartner, the hexahistidine tag and part of the multilinker.
**Figure 3-2****.** Amino Acid sequence of the mTNFH6NS3 clone B9 fusionprotein. Sequence depicted in bold is non-NS3 sequence. This sequence encodes the mTNF fusionpartner, the hexahistidine tag and part of the multilinker.
**Figure 4-1**. DNA coding sequence of the mTNFH6NS3 Type 3a clone 21 fusionprotein. Sequence depicted in bold is non-NS3 sequence. This sequence encodes the mTNF fusionpartner, the hexahistidine tag and part of the multilinker.
**Figure 4-2****.** Amino Acid sequence of the mTNFH6NS3 Type 3a clone 21 fusionprotein. Sequence depicted in bold is non-NS3 sequence. This sequence encodes the mTNF fusionpartner, the hexahistidine tag and part of the multilinker.
**Figure 5-1**. DNA coding sequence of the mTNFH6NS3 Type 3a clone 32 fusionprotein. Sequence depicted in bold is non-NS3 sequence. This sequence encodes the mTNF fusionpartner, the hexahistidine tag and part of the multilinker.
**Figure 5-2****.** Amino Acid sequence of the mTTIFH6NS3 Type 3a clone 32 fusionprotein. Sequence depicted in bold is non-NS3 sequence. This sequence encodes the mTNF fusionpartner, the hexahistidine tag and part of the multilinker.
**Figure 6-1****.** DNA coding sequence of the mTNFH6NS3 Type 2a fusionprotein. Sequence depicted in bold is non-NS3 sequence. This sequence encodes the mTNF fusionpartner, the hexahistidine tag and part of the multilinker.
**Figure 6-2****.** Amino Acid sequence of the mTNFH6NS3 Type 2a fusionprotein. Sequence depicted in bold is non-NS3 sequence. This sequence encodes the mTNF fusionpartner, the hexahistidine tag and part of the multilinker.
**Figure 7-1**. DNA coding sequence of the mTNFH6NS3 Type 2b fusionprotein. Sequence depicted in bold is non-NS3 sequence. This sequence encodes the mTNF fusionpartner, the hexahistidine tag and part of the multilinker.
**Figure 7-2****.** Amino Acid sequence of the mTNFH6NS3 Type 2b fusionprotein. Sequence depicted in bold is non-NS3 sequence. This sequence encodes the mTNF fusionpartner, the hexahistidine tag and part of the multilinker.
**Figure 8-1**. DNA coding sequence of the mTNFH6NS3 Type 2c fusionprotein. Sequence depicted in bold is non-NS3 sequence. This sequence encodes the mTNF fusionpartner, the hexahistidine tag and part of the multilinker.
**Figure 8-2**. Amino Acid sequence of the mTNFH6NS3 Type 2c fusionprotein. Sequence depicted in bold is non-NS3 sequence. This sequence encodes the mTNF fusionpartner, the hexahistidine tag and part of the multilinker.

### EXAMPLES

### Examples 1 to 7, 9 and 10 are for illustrative purposes only.

### Example 1. Expression of HCV NS3 Type 1b clone 19b in E. coli

### 1.1 Cloning of the HCV NS3 Type lb clones 19a and 19b genes

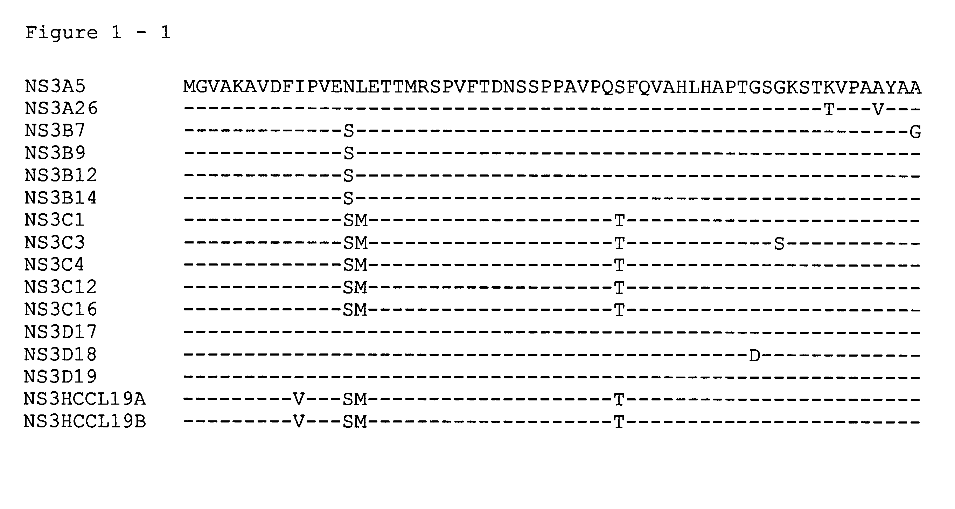

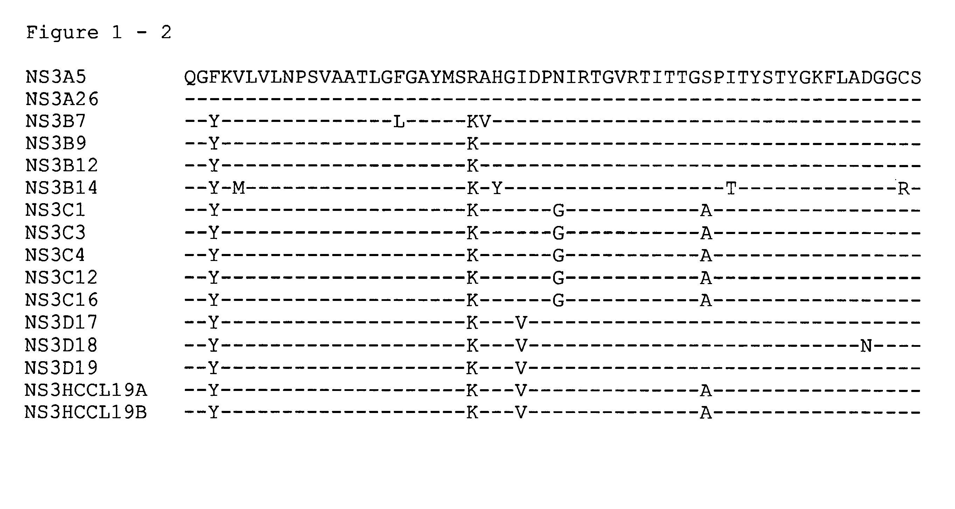

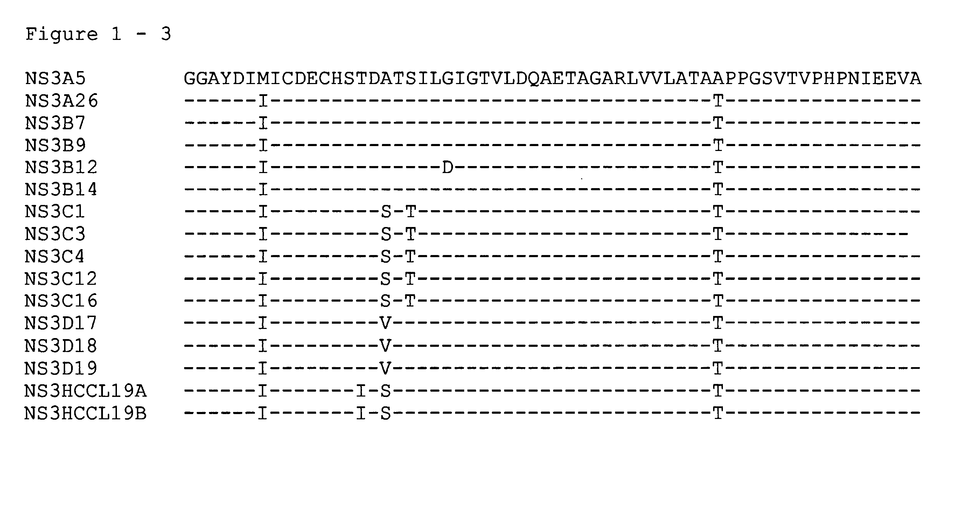

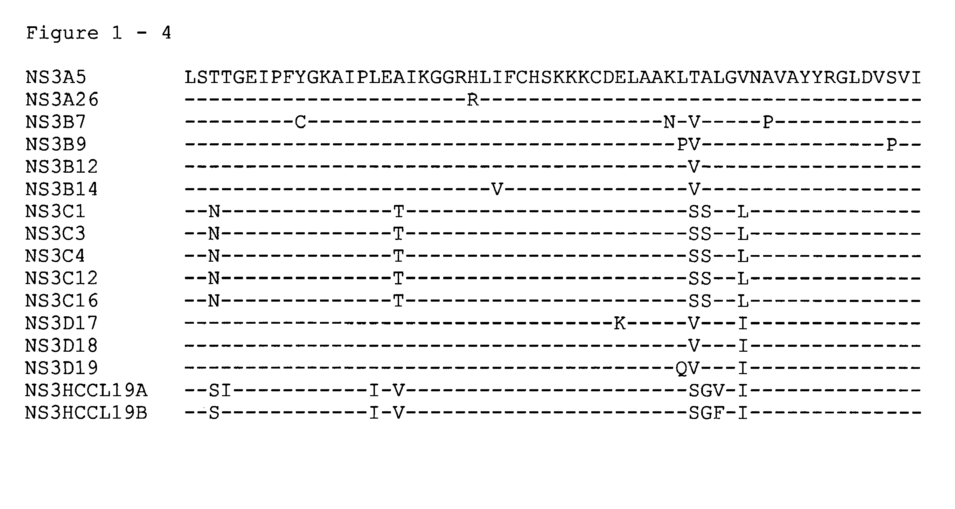

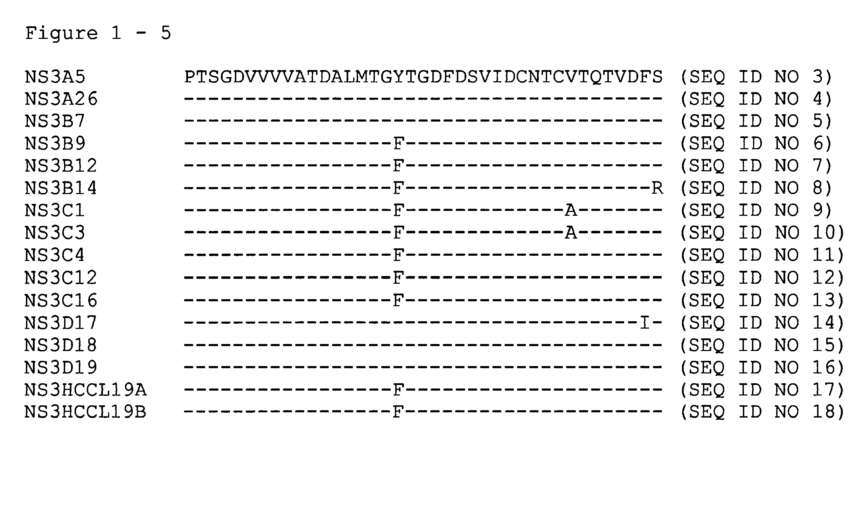

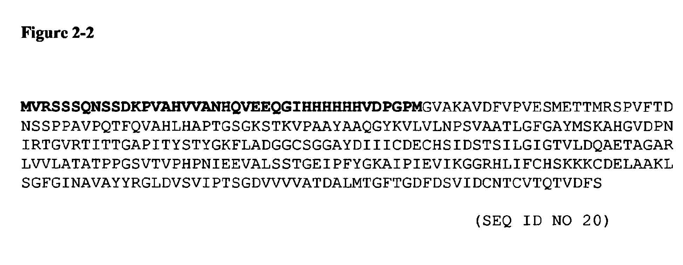

The NS3 helicase domain (amino acids 1188-1465) was amplified by RT-PCR from HCV subtype 1b serum IG8309 (Innogenetics, Ghent, Belgium) using synthetic oligonucleotide primers HCPr59 (5'-GGGCCCCACCATGGGGGTTGCGAAGGCGGTGGACTT-3') (SEQ ID NO 1) and HCPr60 (5'-CTATTAGCTGAAAGTCGACTGTCTGGGTGACAGCA-3') (SEQ ID NO 2). This yielded a PCR fragment 19 which was cloned into *E*. *coli.* The sense primer HCPr59 introduces an Apal restriction site which includes an artifical methionine. Antisense oligonucleotide HCPr60 introduces a stopcodon after aa 1465. The PCR fragment was subsequently cut with Apal and the resulting 833 bp ApaI fragment was cloned in the ApaI-cut expressionvector pmTNFHRP (Innogenetics, Ghent, Belgium). Four hepatitis C clones (HCCl) were sequenced: HCC119a and HCC119b (see deduced amino acid sequence given in Figure 1 and Figure 2-2). Clone HCC119b (pmTNFHRPHCC119b) was retained for further subcloning.

### 1.2 Construction of the expression plasmid pEmTNFMPHHCCl19b

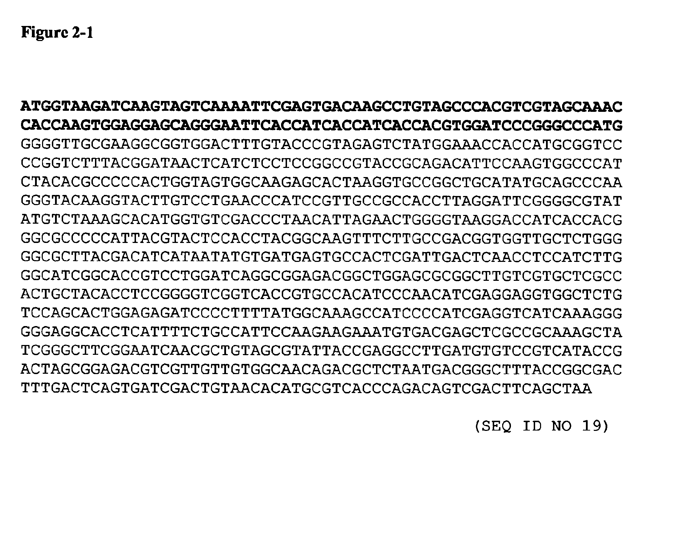

Starting from vector pmTNFHRPHCCl19b the NS3 clone 19b coding sequence was isolated as a 900 bp NcoI fragment and inserted into the NcoI-cut expressionvector pEmTNFMPH (Innogenetics, Ghent, Belgium) resulting in vector pEmTNFMPHHCCl19b. This plasmid expresses HCV NS3 clone 19b as an N-terminal fusionprotein with the N-terminal 25 aa of murine TNF followed by a hexahistidine purification tag and a formic acid cleavage site (SEQ ID NOs 19 and 20; Figure 2).

### 1.3 Expression of HCV NS3 clone 19b in E.coli

*E. coli* strain MC1061(pAcI) cells (Wertman et al., 1986) were transformed with plasmid pEmTNFMPHHCCl19b. MC1061(pAcI) cells harboring pEmTNFMPHHCCl19b were grown overnight in Luria Broth (LB) supplemented with 10 µg/ml tetracycline at 28°C. Cultures were diluted 20 times in fresh LB, then grown at 28°C to an OD₆₀₀ of 0.2, after which the temperature was raised to 42°C. At 2 to 3 hours post-induction, the cells were harvested. Expression of the HCV NS3 clone 19b fusion protein was analysed by western blotting using specific monoclonal antibodies and HCV positive human sera.

### Example 2. Expression of HCV NS3 clone B9 in E.coli

### 2.1 Cloning of the HCV NS3 Type 1a clone B9 gene

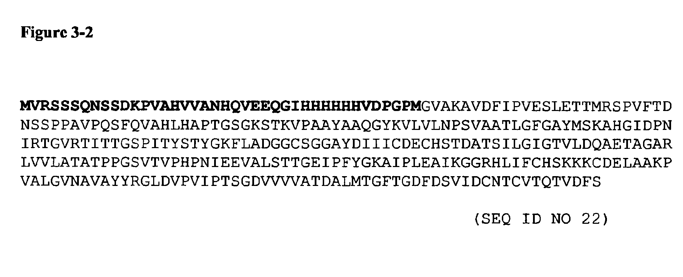

The NS3 helicase domain (amino acids 1188-1465) was amplified by RT-PCR from HCV subtype 1a serum IG21054 (Innogenetics, Ghent, Belgium) using synthetic oligonucleotide primers HCPr59 (5'-GGGCCCCACCATGGGGGTTGCGAAGGCGGTGGACTT-3') (SEQ ID NO 1) and HCPr60 (5'-CTATTAGCTGAAAGTCGACTGTCTGGGTGACAGCA-3') (SEQ ID NO 2). This yielded a PCR fragment B which was cloned into *E. coli.* The sense primer HCPr59 introduces an ApaI restriction site which includes an artifical methionine. Antisense oligonucleotide HCPr60 introduces a stopcodon after aa 1465. The PCR fragment was subsequently cloned in the pGEM-T vector (Promega, Madison, WI, US). Four clones were sequenced: B7, B9, B12, and B 14 (see deduced amino acid sequences in Figure 1 and Figure 3-2). Clone B9 (pGEMTNS3B9) was retained for further subcloning.

### 2.2 Construction of the expressionplasmid pIGFH111NS3B9

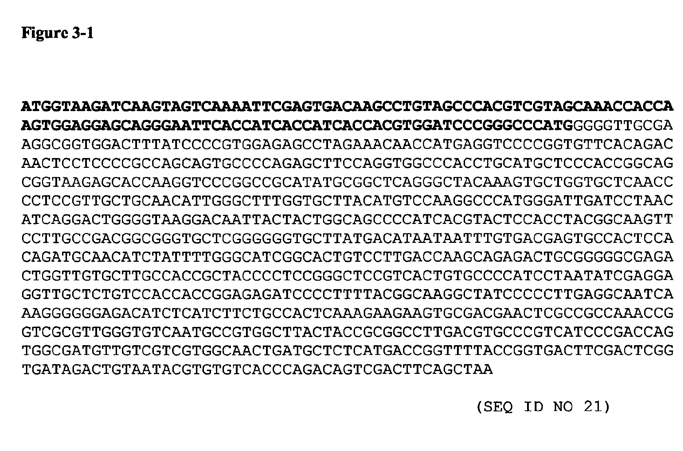

Starting from vector pGEMTNS3B9, the clone B9 coding sequence was isolated as a 850 bp NcoI/SpeI blunted fragment and inserted into the NcoI/Stul cut expression vector pIGFH111 (Innogenetics, Ghent, Belgium) resulting in vector pIGFH111NS3B9. This plasmid expresses HCV NS3 clone B9 as an N-terminal fusion protein with the N-terminal 25 aa of murine TNF followed by a hexahistidine purification tag and a formic acid cleavage site (SEQ ID NOs. 21 and 22; Figure 3).

### 2.3 Expression of HCV NS3 clone B9 in E.coli

*E.coli* strain MC1061(pAcl) (Wertman et al., 1986) cells were transformed with plasmid pIGFH111NS3B9. MC1061(pAcI) cells harboring pIGFH111NS3B9 were grown overnight in Luria Broth (LB) supplemented with 10 µg/ml tetracycline at 28°C. Cultures were diluted 20 times in fresh LB, then grown at 28°C to an OD₆₀₀ of 0.2, after which the temperature was raised to 42°C. At 2 to 3 hours post-induction, the cells were harvested. Expression of the HCV NS3 clone B9 fusion protein was analysed on Western blot using specific monoclonal antibodies and HCV positive human sera.

### Example 3. Expression of HCV NS3 Type 1a clones A26, C16, and D18 in E.coli

Clones A26, C16, and D18 were isolated from HCV subtype 1a infected sera IG21051, IG17790, and IG21068, respectively, in a similar way as described for clone B9 using primers HCPr59 and HCPr60. Initially, clones, A5, A26, C1, C3, C4, C12, C16, D17, D18, and D19, were cloned and sequenced (see deduced amino acid sequences given in Figure 1). Clones A26, C16, and D18 were retained for further subcloning.

### Example 4. Expression of HCV NS3 Type 3a clones 21 and 32 in E.coli

### 4.1 Cloning of the HCV NS3 Type 3a clones 21 and 32 genes

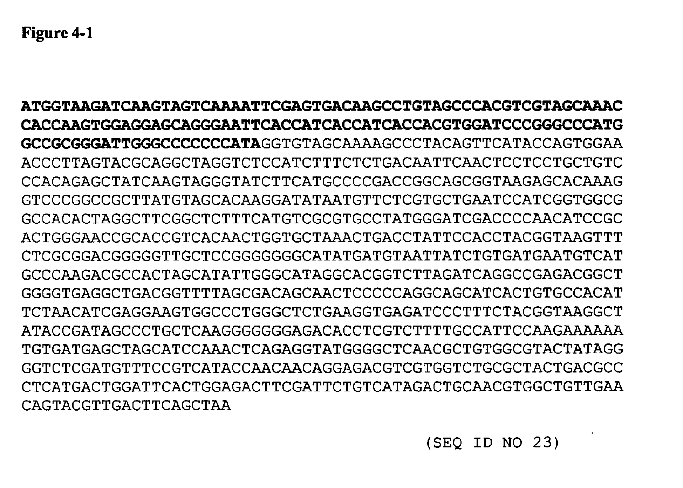

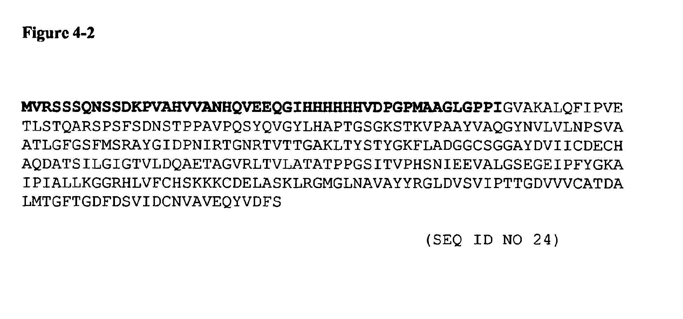

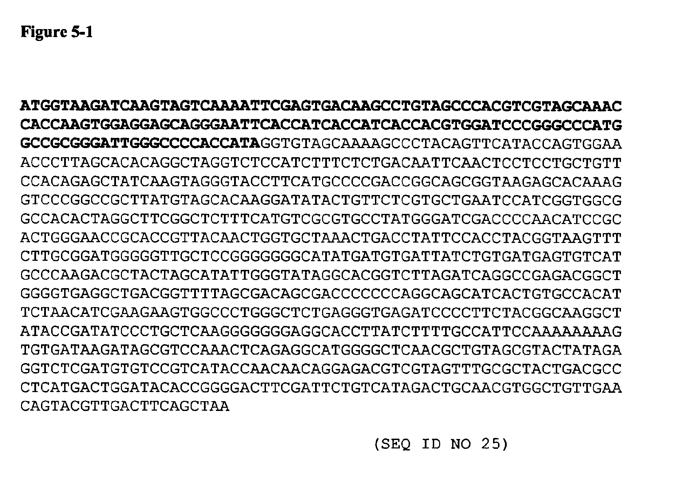

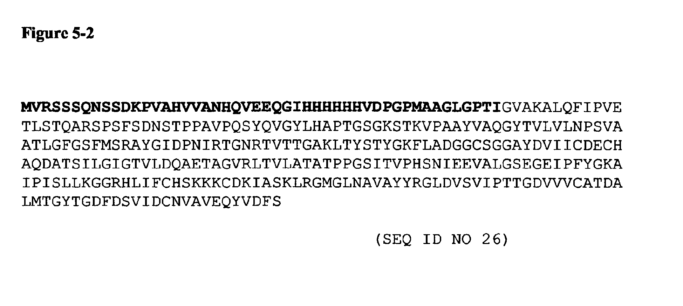

The NS3 helicase domain (amino acids 1188-1465) was amplified by RT-PCR from HCV subtype 3a sera IG21349 and IG20014 (Innogenetics, Ghent, Belgium) using synthetic oligonucleotide primers 403 (5'GGGCCCCACCATAGGTGTAGCAAAAGCCCTACAGTT-3') (SEQ ID NO 33) and 404 (5'-CTATTAGCTGAAGTCAACGTACTGTTCAACAGC-3') (SEQ ID NO 34). This yielded in both cases a PCR fragment of approx. 850 bp which was subsequently subcloned in the pGEM-T vector (Promega, Madison, WI, US). From each cloned PCR fragment several clones were sequenced but from each serum only one cloned fragment proved to be completely correct upon sequencing. This was clone 21 (pGEM-TNS3T3a.21) for serum IG21349 and clone 32 (pGEM-TNS3T3a.32) for serum IG20014 (Figures 4 and 5).

### 4.2 Construction of the expressionplasmids pIGFHlllNS3T3a.21 and pIGFIi111NS3T3a.32

Starting from vectors pGEM-TNS3T3a.21 and pGEM-TNS3T3a.32, the clone 21 and 32 coding sequences were isolated as 850 bp NcoI/SalI fragments and inserted into the NcoI/SalI cut expression vector pIGFH111 (Innogenetics, Ghent, Belgium) resulting in vectors pIGFH111NS3T3a.21 and pIGFHIIINS3T3a.32, respectively. These plasmids express HCV NS3 Type 3a clones 21 and 32 as N-terminal fusion proteins with the N-terminal 25 aa of murine TNF followed by a hexahistidine purification tag and a formic acid cleavage site (SEQ ID NOs 23-26; Figures 4 and 5).

### 4.3 Expression of HCV NS-3 Type 3a clones 21 and 32 in E.coli

*E.coli* strain MC1061(pAcI) (Wertman et al., 1986) cells were transformed with plasmids pIGFH111NS3T3a.21 and pIGFH111NS3T3a.32, respectively. MC1061(pAcI) cells harboring pIGFH111NS3T3a.21 or pIGFH111NS3T3a.32 were grown overnight in Luria Broth (LB) supplemented with 10 g/ml tetracycline at 28 C. Cultures were diluted 20 times in fresh LB, then grown at 28 C to an OD600 of 0.2, after which the temperature was raised to 42 C. At 2 to 3 hours post-induction, the cells were harvested. Expression of the HCV NS3 Type 3a clones 21 and 32 fusionproteins was analysed on Western blot using specific monoclonal antibodies and HCV positive human sera.

### Example 5. Expression of HCV NS3 Type 2a clone 3 in E.coli

### 5.1 Cloning of the HCV NS3 Type 2a clone 3 gene

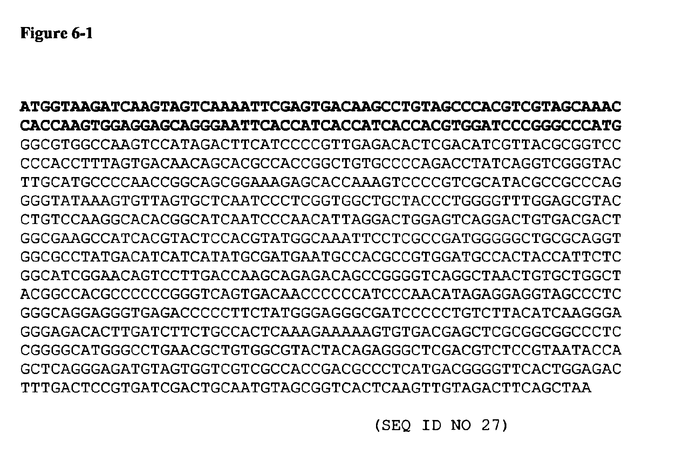

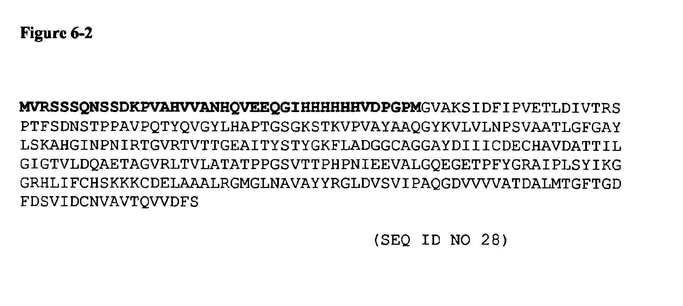

The NS3 helicase domain (amino acids 1188-1465) was amplified by RT-PCR from a HCV subtype 2a serum IG21342 (Innogenetics, Ghent, Belgium) using synthetic oligonucleotide primers 412 (5'-GGGCCCCACCATGGGCGTGGCCAAGTCCATAGACTT-3') (SEQ ID NO 35) and 413 (5'-CTATTAGCTGAAGTCTACAACTTGAGTGACCGC-3') (SEQ ID NO 36). This yields a PCR fragment of approx. 850 bp which was subsequently subcloned in the pGEM-T vector (Promega, Madison, WI, US). Several clones were sequenced and clone 3 (pGEM-TNS3T2a) was retained for further subcloning (Figure 6).

### 5.2 Construction of expressionplasmid pIGFH111NS3T2a

Starting from vector pGEM-TNS3T2a, the clone 3 coding sequence was isolated as a 850 bp NcoI fragment and inserted into the Ncol cut expression vector pIGFH111 (Innogenetics, Ghent, Belgium) resulting in vector plGFH111NS3T2a. This plasmid expresses HCV NS3 Type 2a clone 3 as an N-terminal fusion protein with the N-terminal 25 aa of murine TNF followed by a hexahistidine purification tag and a formic acid cleavage site (SEQ ID NOs 27 and 28; Figure 6).

### 5.3 Expression of HCV NS-3 Type 2a clone 3 in E.coli

*E. coli* strain MC1061(pAcI) (Wertman et al., 1986) cells were transformed with plasmid pIGFH111NS3T2a. MC1061(pAcI) cells harbouring plGFH111NS3T2a were grown overnight in Luria Broth (LB) supplemented with 10 µg/ml tetracycline at 28°C. Cultures were diluted 20 times in fresh LB, then grown at 28°C to an OD600 of 0.2, after which the temperature was raised to 42°C. At 2 to 3 hours post-induction, the cells were harvested Expression of the HCV NS3 Type 2a clone 3 fusionprotein was analysed on Western blot using specific monoclonal antibodies and HCV positive human sera.

### Example 6. Expression of HCV NS3 Type 2b clone 9 in E.coli

### 6.1 Cloning of the HCV NS3 Type 2b clone 9 gene

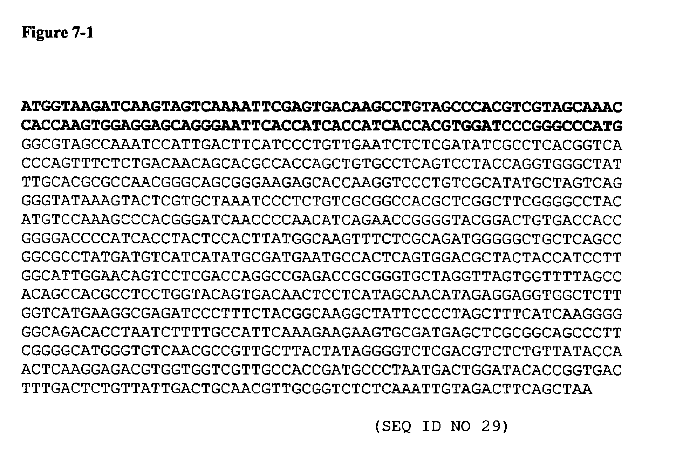

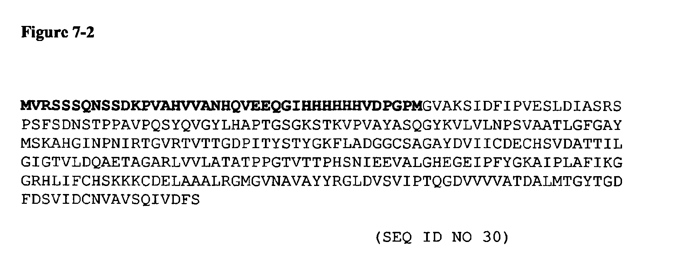

The NS3 helicase domain (amino acids 1188-1465) was amplified by RT-PCR from a HCV subtype 2b serum IG20192 (Innogenetics, Ghent, Belgium) using synthetic oligonucleotide primers 401 (5'-GGGCCCCACCATGGGCGTAGCCAAATCCATTGACTT-3') (SEQ ID NO 37) and 402 (5'-CTATTAGCTGAAGTCTACAATTTGAGAGACCGC-3') (SEQ ID NO 38). This yields a PCR fragment of approx. 850 bp which was subsequently subcloned in the pGEM-T vector (Promega, Madison, WI, US). Several clones were sequenced and clone 9 was retained for further subcloning (Figure 7).

### 6.2 Construction of expression plasmid pIGFH111NS3T2b

Starting from vector pGEM-TNS3T2b, the clone 9 coding sequence was isolated as a 850 bp NcoI fragment and inserted into the NcoI cut expression vector pIGFH111 (Innogenetics, Ghent, Belgium) resulting in vector pIGFH111NS3T2b. This plasmid expresses HCV NS3 Type 2b clone 9 as an N-terminal fusion protein with the N-terminal 25 aa of murine TNF followed by a hexahistidine purification tag and a formic acid cleavage site (SEQ ID NOs 29-30; Figure 7).

### 6.3 Expression of HCV NS-3 Type 2b clone 9 in E.coli

*E. coli* strain MC 1061 (pAcI) cells (Wertman et al., 1986) were transformed with plasmid pIGFH111NS3T2b. MC1061 (pAcI) cells harbouring pIGFHIIINS3T2b were grown overnight in Luria Broth (LB) supplemented with 10 µg/ml tetracycline at 28°C. Cultures were diluted 20 times in fresh LB, then grown at 28°C to an OD600 of 0.2, after which the temperature was raised to 42°C. At 2 to 3 hours post-induction, the cells were harvested. Expression of the HCV NS3 Type 2b clone 9 fusionprotein was analysed on Western blot using specific monoclonal antibodies and HCV positive human sera.

### Example 7. Expression of HCV NS3 Type 2c clone 14 in E.coli

### 7.1 Cloning of the HCV NS3 Type 2c clone 14 gene

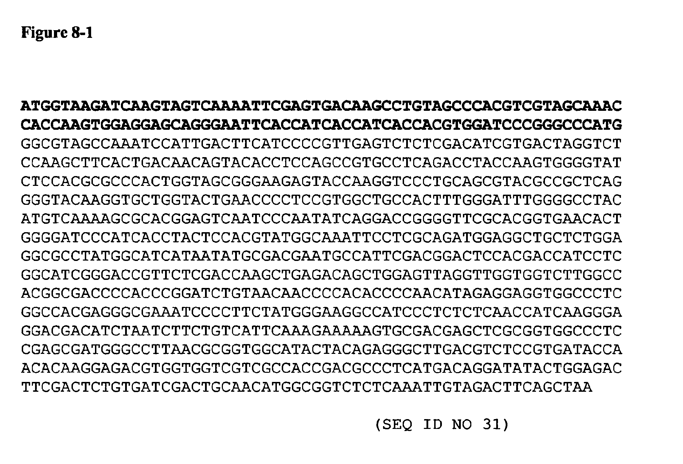

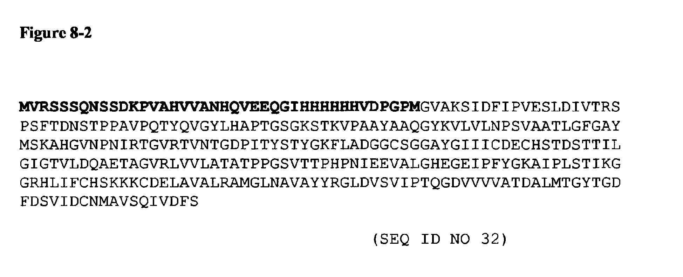

The NS3 helicase domain (amino acids 1188-1465) was amplified by RT-PCR from a HCV subtype 2c serum IG20031 (Innogenetics, Ghent, Belgium) using synthetic oligonucleotide primers 401 (5'-GGGCCCCACCATGGGCGTAGCCAAATCCATTGACTT-3') (SEQ ID NO 37) and 402 (5'-CTATTAGCTGAAGTCTACAATTTGAGAGACCGC-3') (SEQ ID NO 38). This yields a PCR fragment of approx. 850 bp which was subsequently subcloned in the pGEM-T vector (Promega, Madison, WI, US). Several clones were sequenced and clone 14 (pGEM-TNS3T2c) was retained for further subcloning (Figure 8).

### 7.2 Construction of expressionplasmid pIGFH111NS3T2c

Starting from vector pGEM-TNS3T2c, the clone 14 coding sequence was isolated as a 850 bp NcoI fragment and inserted into the NcoI cut expression vector pIGFH111 (Innogenetics, Ghent, Belgium) resulting in vector pIGFH111NS3T2c. This plasmid expresses HCV NS3 Type 2c clone 14 as an N-terminal fusion protein with the N-terminal 25 aa of murine TNF followed by a hexahistidine purification tag and a formic acid cleavage site (SEQ ID NOs 31 and 32; Figure 8).

### 7.3 Expression of HCV NS-3 Type 2c clone 14 in E.coli

E.coli strain MC1061(pAcI) cells (Wertman et al., 1986) were transformed with plasmid pIGFH111NS3T2c. MC1061(pAcI) cells harbouring pIGFH111NS3T2c were grown overnight in Luria Broth (LB) supplemented with 10 µg/ml tetracycline at 28°C. Cultures were diluted 20 times in fresh LB, then grown at 28°C to an OD600 of 0.2, after which the temperature was raised to 42°C. At 2 to 3 hours post-induction, the cells were harvested. Expression of the HCV NS3 Type 2c clone 14 fusionprotein was analysed on Western blot using specific monoclonal antibodies and HCV positive human sera.

### Example 8. Purification of the NS3 helicase protein domain

Nine volumes of 8M Guanidinium hydrochloride (Gu.HCl) and 1 volume of 0.2 M NaHPO₄ were added to each gram equivalent of wet *E*. *coli* cell paste and the solution was homogenized by continuously vortexing. Solid Na₂S₄O₆ and Na₂SO₃ were added to the solution up to a final concentration of 65 and 360 mM, respectively. CuSO₄ (stock solution: 0.1 M in 25% NH₃) was added up to a final concentration of 100µM. The solution was stirred overnight in the dark at room temperature and after incubation at -70°C cleared by centrifugation at 4°C (30 min, 20.000 rpm, JA20 rotor).
Empigen BB^{™} (Albright & Wilson Ltd., Okibury, UK) and imidazole were added to the supernatant up to a final concentration of 1% (w/v) and 20 mM, respectively. The pH was adjusted to 7.2 with IN HCl. A sample corresponding to 3 L cell culture equivalent was loaded at 2 mL/min on a 25 mL Ni-IDA Sepharose FF (XK 16/20 column, Pharmacia, Upsala, Sweden), which had been equilibrated with buffer A containing 20 mM imidazole (buffer A: 50 mM phosphate, 6M Gu.HCl, 1% Empigen, pH 7.2). The Ni-IDA Sepharose column was washed consecutively with:
- buffer A containing 20mM imidazole
- buffer A containing 35 mM imidazole
- buffer A containing 50 mM imidazole
- buffer B containing 50 mM imidazole (buffer B: 50 mM phosphate, 6M Gu.HCl, pH 7.2)
- buffer B containing 200 mM imidazole.
Each washing step was maintained during the chromatography untill the absorbance at 280 nm reached baseline level. The column was regenerated with 50 mM EDTA, 500 mM NaCl, pH 7.0.

Fractions were analysed by SDS-PAGE using non-reducing conditions and silver staining. The mTNF-NS3 B9 fusion protein was recovered in the 200 mM imidazole elution. Western blotting using rabbit anti-human TNF (1µg NS3/lane) and rabbit anti-*E*. *coli* (10 µg NS3/lane) showed that the NS3 exhibited a purity of over 99 % after this single chromatography step.

The 200 mM imidazole elution fractions were pooled and desalted.

A 40 mL Ni-IDA eluate sample was loaded at 10 mL /min on a 300 mL Sephadex G25 column (XK 50, Pharmacia, Upsala, Sweden) which had been equilibrated with 50 mM phosphate, 6M ureum, 1mM EDTA, pH 7.2. 10 mL-fractions were collected and the protein concentration was determined by the micro BCA method (Pierce, Rockford, IL, US). The protein concentration was adjusted to 500 µg/mL with the desalting buffer before desulphonation and reduction. The overall yield was 50-55 mg purified NS3 fusion protein/L culture equivalent.

Finally, DTT (stock solution: 100 mM in destilled water) was added in a 100-fold molar excess versus the cysteine content in the NS3 antigen (e.g. NS3 19b contains 7 cysteins). The solution was flushed with nitrogen and incubated for 1h at 28°C. The NS3 sample was subsequently diluted in the appropiate buffer for ELISA and LIA coating.

### Example 9. NS3 helicase antibody reactivity tested in LIA

In order to test the NS3 helicase antibody reactivity, a line of 50 µg/ml NS3 antigen solution in phosphate buffered saline was applied onto nylon membrane strips. The strips were dried for at least 1 hour at a temperature between 18-24°C and were subsequently blocked with PBS/caseine in the presence (10 mM) or absence of the reducing agent DTT. The strips were subsequently washed with PBS containing Tween 20 and either no DTT or 10 mM DTT and with water containing either no DTT or 10 mM DTT and 1 mM EDTA. The membranes were dried for 30 minutes and cut into strips for testing of different patient samples.

The results of an experiment wherein strips were incubated with the anti-HCV seroconversion panel PHV903 (Boston Biomedica Inc., Boston, US) are given in Table 1.

### Example 10. NS3 helicase antibody reactivity tested in ELISA

In order to test the NS3 helicase antibody reactivity, ELISA plates were coated with the NS3 antigens purified as in Example 4 in the following way.

Microtiter plate wells were coated with NS3 protein at a concentration of 0.3 µg/ml NS3 protein in coating buffer containing 50 mM carbonate buffer, either 200 mM DTT or no DTT, and 1 mM EDTA. The microtiter plates are incubated for 18 hours at 20° C, and blocked with 300 µl of PBS/caseine buffer per well. The plates were incubated for 2 hours at 20°C and subsequently fixed with 300 µl of fixation buffer containing either 200 mM DTT or no DTT, and 1 mM EDTA for 2 hours at 20°C.

The results are shown in Tables 2 and 3. Table 2 gives the Signal to Noise values of assays including NS3 coated and fixed with or without DTT, with the BBI seroconversion panels PHV901 to PHV912. Table 3 shows a summary of the number of days in which HCV antibodies can be detected earlier by the assay incorporating DTT. Clearly, a total number of 34 days of earlier detection in 12 HCV seroconversions can be obtained by incorporating DTT in the assay.

**Table 1. BBI panels tested in LIA coated with HCV NS3 as described in example 9.**

| **PHV** | **+DTT¹** | **-DTT**¹ |
|---|---|---|
| 903-01 | - | - |
| 903-02 | - | - |
| 903-03 | +/- | - |
| 903-04 | 2 | - |
| 903-05 | 2 | +/- |
| 903-06 | 2 | +/- |
| 903-07 | 4 | 2 |
| 903-08 | 4 | 2 |

| | | |
|---|---|---|
| ¹-: no reaction; +positive reaction; intensity ratings are given in comparison with different cut off lines sprayed onto the same strip. | | |

**Table 2: BBI panels tested in ELISA coated with HCV NS3 as described in example 10.**

| **MEMBER ID#** | **BLEED DATE** | **+ DTT (OD₄₅₀)** | **- DTT (OD₄₅₀)** |
|---|---|---|---|
| PHV901-01 | 09/23/93 | 0.1 | 0.3 |
| PHV901-02 | 11/27/93 | 0.1 | 0.3 |
| PHV901-03 | 12/29/93 | 2.0 | 2.9 |
| PHV901-04 | 12/31/93 | 2.1 | 3.0 |
| PHV901-05 | 01/05/94 | 2.2 | 3.1 |
| PHV901-06 | 01/07/94 | 2.4 | 3.2 |
| PHV901-07 | 02/01/94 | 4.1 | 6.0 |
| PHV901-08 | 02/09/94 | 3.9 | 5.9 |
| PHV901-09 | 03/01/94 | 4.0 | 7.9 |
| PHV901-10 | 03/08/94 | 4.1 | 7.8 |
| PHV901-11 | 04/14/94 | 4.2 | 8.3 |
| | | | |
| PHV903-01 | 02/07/92 | 0.2 | 0.2 |
| PHV903-02 | 02/12/92 | 0.9 | 0.9 |
| PHV903-03 | 02/14/92 | 1.3 | 1.6 |
| PHV903-04 | 02/19/92 | 2.5 | 2.7 |
| PHV903-05 | 02/21/92 | 2.8 | 2.8 |
| PHV903-06 | 02/26/92 | 3.2 | 4.6 |
| PHV903-07 | 02/28/92 | 3.5 | 5.4 |
| PHV903-08 | 03/04/92 | 3.5 | 4.1 |
| | | | |
| PHV904-01 | 04/18/95 | 0.1 | 0.2 |
| PHV904-02 | 04/20/95 | 0.1 | 0.3 |
| PHV904-03 | 04/25/95 | 0.1 | 0.2 |
| PHV904-04 | 04/27/95 | 0.1 | 0.2 |
| PHV904-05 | 05/02/95 | 0.4 | 0.4 |
| PHV904-06 | 05/09/95 | 0.8 | 0.5 |
| PHV904-07 | 05/11/95 | 0.8 | 0.5 |
| | | | |
| PHV905-01 | 11/17/95 | 0.1 | 0.2 |
| PHV905-02 | 11/21/95 | 0.1 | 0.3 |
| PHV905-03 | 11/24/95 | 0.1 | 0.3 |
| PHV905-04 | 11/28/95 | 0.2 | 0.3 |
| PHV905-05 | 12/01/95 | 0.5 | 0.3 |
| PHV905-06 | 12/05/95 | 1.0 | 0.4 |
| PHV905-07 | 12/08/95 | 2.5 | 0.8 |
| PHV905-08 | 12/12/95 | 3.5 | 2.2 |
| PHV905-09 | 12/15/95 | 3.5 | 3.2 |

| **MEMBER ID#** | **BLEED DATE** | **+ DTT** | **- DTT** |
|---|---|---|---|
| PHV 907-01 | 04/06/96 | 0.1 | 0.2 |
| PHV907-02 | 04/10/96 | 0.1 | 0.2 |
| PHV907-03 | 04/13/96 | 0.1 | 0.2 |
| PHV907-04 | 04/19/96 | 3.0 | 2.2 |
| PHV907-05 | 04/24/96 | 3.7 | 4.1 |
| PHV907-06 | 04/27/96 | 3.6 | 4.1 |
| PHV907-07 | 09/17/96 | 3.9 | 7.6 |
| | | | |
| PHV908-01 | 01/26/96 | 0.1 | 0.1 |
| PHV908-02 | 01/29/96 | 0.1 | 0.1 |
| PHV908-03 | 01/31/96 | 0.1 | 0.1 |
| PHV908-04 | 02/06/96 | 0.1 | 0.1 |
| PHV908-05 | 02/08/96 | 0.1 | 0.1 |
| PHV908-06 | 02/14/96 | 0.2 | 0.1 |
| PHV908-07 | 02/20/96 | 1.4 | 0.2 |
| PHV908-08 | 02/22/96 | 1.6 | 0.2 |
| PHV908-09 | 02/27/96 | 1.9 | 0.2 |
| PHV908-10 | 03/01/96 | 2.3 | 0.2 |
| PHV908-11 | 03/07/96 | 2.3 | 0.4 |
| PHV908-12 | 03/11/96 | 2.8 | 0.5 |
| PHV908-13 | 03/14/96 | 2.8 | 0.5 |
| | | | |
| PHV909-01 | 01/28/96 | 0.1 | 0.4 |
| PHV909-02 | 02/15/96 | 2.3 | 5.4 |
| PHV909-03 | 02/17/96 | 2.4 | 5.3 |
| | | | |
| PHV910-01 | 08/26/96 | 0.1 | 0.2 |
| PHV910-02 | 08/30/96 | 0.4 | 0.2 |
| PHV910-03 | 09/03/96 | 2.7 | 3.1 |
| PHV910-04 | 09/06/96 | 3.6 | 6.4 |
| PHV910-05 | 09/10/96 | 3.9 | 8.1 |
| | | | |
| PHV911-01 | 10/30/96 | 0.1 | 0.2 |
| PHV911-02 | 11/02/96 | 0.1 | 0.2 |
| PHV911-03 | 11/13/96 | 2.1 | 4.0 |
| PHV911-04 | 11/20/96 | 3.6 | 7.8 |
| PHV911-05 | 11/23/96 | 3.7 | 7.7 |
| | | | |
| PHV912-01 | 01/06/96 | 0.2 | 0.3 |
| PHV912-02 | 01/10/96 | 0.2 | 0.2 |
| PHV912-03 | 01/13/96 | 4.5 | 9.9 |
| | | | |

| **MEMBER ID#** | **BLEED DATE** | **+DTT** | **-DTT** |
|---|---|---|---|
| PHV902-01 | 02/10/92 | 0.1 | 0.2 |
| PHV902-02 | 02/12/92 | 0.1 | 0.2 |
| PHV902-03 | 02/17/92 | 0.1 | 0.3 |
| PHV902-04 | 02/19/92 | 0.3 | 0.6 |
| PHV902-05 | 02/24/92 | 2.6 | 3.9 |
| PHV902-06 | 02/26/92 | 3.1 | 5.9 |
| PHV902-07 | 03/02/92 | 3.4 | 6.5 |
| | | | |
| PHV906-01 | 10/07/95 | 0.5 | 0.3 |
| PHV906-02 | 10/09/95 | 0.5 | 0.4 |
| PHV906-03 | 10/14/95 | 1.6 | 0.6 |
| PHV906-04 | 10/17/95 | 1.5 | 1.2 |
| PHV906-05 | 10/21/95 | 2.2 | 3.0 |
| PHV906-06 | 10/24/95 | 2.5 | 4.5 |
| PHV906-07 | 10/28/95 | 2.9 | 5.7 |

**Table 3. Overview of the BBI panels - numbers of days with earlier detection**

| **PHV** | **+DTT** | **-DTT** |
|---|---|---|
| 901 | 0 | 0 |
| 902 | 0 | 0 |
| 903 | 0 | 0 |
| 904 | 0 | 0 |
| 905 | 7 | 0 |
| 906 | 3 | 0 |
| 907 | 0 | 0 |
| 908 | 24 | 0 |
| 910 | 0 | 0 |
| 911 | 0 | 0 |
| 912 | 0 | 0 |

### REFERENCES

Burns, J., Butler, J., Moran, J., and Whitesides, G. (1991) Selective reduction of disulfides by tris(2-carboxyethyl)phosphine. J. Org. Chem. 56, 2648-2650.
Chan, W. (1968) A method for the complete S sulfonation of cysteine residues in proteins. Biochemistry 7, 4247-4254.
Claeys, H., Volkaerts, A., Verhaert, H., De Beenhouwer, H., and Vermylen, C. (1992) Evaluation of anti-HCV capsid indeterminate samples. The Lancet 340, 249.
Cole, R. (1967) Sulfitolysis. Meth. Enzymol. 11, 206.
Coligan, J., Kruisbeek, A., Margulis, D., Shevach, E. and Strober, W. (1992) Current protocols in immunology. Wiley Interscience.
De Beenhouwer, H., Verhaert, H., Claeys, H., and Vermylen, C. (1992) Confirmation of hepatitis C virus positive blood donors by immunoblotting and polymerase chain reaction. Vox. Sang. 63, 198-203.
Di Bisceglie, AM, Carithers, RL Jr, Gores, GJ (1998) Hepatocellular carcinoma. Hepatology. 28, 1161-1165.
Gailit, J. (1993) Restoring free sulfhydryl groups in synthetic peptides. Anal. Biochem., 214, 334-335.
Holmgren, A. (1979) Thioredoxin catalyzes the reduction of insulin disulfides by dithiothreitol and dihydrolipoamide. J. Biol. Chem. 254, 9627-9632.
Inglis, A., and Liu, T. (1970) The stability of cysteine and cystine during acid hydrolysis of proteins and peptides. J. Biol. Chem. 245, 112-116.
McFarlane, I., Smith, H., Johnson, P., Bray, G., Vergani, D., and Williams, R. (1990) Hepatitis C virus antibodies in chronic active hepatitis: pathogenic factor or false-positive result? The Lancet 335, 754-757.
Maertens, G. and Stuyver, L. (1997) Genotypes and Genetic variation of hepatitis C virus. In: Molecular Medicine of Hepatitis (Eds. Zuckerman, A. and Harrison, T.), Molecular Medical Science Series (Eds. James, K. and Morris A) John Wiley and Sons Ltd., Chichester, England, Chapter 13, pp. 183-233.
Marin, M., Bresciani, S., Puoti, M., Rodella, A., Gussago, A., Ravaggi, A., Pizzocolo, G., Albertini, A., and Cariani, E. (1994) Clinical significance of serum HCV RNA as marker of HCV infection. J. Clin. Microbiol. 32, 3008-3012.
Peeters, D., Dekeyser, F., DeLeys, R., Maertens, G., and Pollet, D. (1993) Confirmation of anti-hepatitis C virus antibodies using the INNO-LIA HCV Ab III including Core, E2/NS1, NS3, NS4, and NS5 epitopes. International Symposium on Viral Hepatitis and Liver Disease, Tokyo, abstract 413.
Pollet, D., Saman, E., Peeters, D., Warmenbol, H., Heyndricks, L., Wouters, C., Beelaert, G., van der Groen, G., and Van Heuverswyn, H. (1990) Confirmation and differentiation of antibodies to human immunodeficiency virus 1 and 2 with a strip-based assay including recombinant antigens and synthetic peptides. Clin. Chem. 37, 1700-1707.
Saito, I., Miyamura, T., Ohbayashi, A., Harada, H., Katayama, T., Kikuchi, S., Watanabe, Y., Koi, S., Onji, M., Ohta, Y., Choo, Q.-L., Houghton, M., and Kuo, G. (1990) Proc. Natl. Acad. Sci. USA 87, 6547-6549.
Singh, R., and Kats, L. (1995) Catalysis of reduction of disulfide by selenol. Anal. Biochem.,232, 86-91.
Stuyver, L., Fretz, C., Esquivel, C., Boudifa, A., Jaulmes, D., Azar, N., Lunel, F., Leroux-Roels, G., Maertens, G., and Fournel, J. (1996) HCV genotype analysis in apparently healthy anti-HCV positive Parisian blood donors. Transfusion 36, 552-558.
Thakur, M., DeFulvio, J., Richard, M., and Park, C. (1991) Technetium-99m labeled monoclonal antibodies: evaluation of reducing agents. Nucl. Med. Biol., 18, 227- 233.
Waumans, L., Claeys, H., Verhaert, H., Mertens, W., and Vermylen, C. (1993) Hepatitis C virus confirmation in blood donor screening. Vox. Sang. 64, 145-149.
Wertman K.F., Wyman A.R. and Botstein D. (1986) Host/vector interactions which affect the viability of recombinant phage lambda clones. Gene 49: 253-262.
Zaaijer, H., Vrielink, H., van Exel-Oehlers, P., Cuypers, H., and Lelie, P. (1994) Confirmation of hepatitis C infection: a comparison of five immunoblot assays. Transfusion 34, 603-607.

## Claims

1. A method for purifying a cysteine containing recombinantly expressed HCV NS3 protein comprising steps (a) and (c), and at least 1 of the following steps (b), (d) or (e):
(a) sulphonation of a lysate from recombinant host cells,
(b) treatment with a zwitterionic detergent, preferably after removal of the cell debris,
(c) purification of the sulphonated version of the recombinant protein or purification of the sulphonated version of the recombinant protein with subsequent removal of the detergent,
(d) desulphonation of the sulphonated recombinant protein,
(e) storage in the presence of a molar excess of DTT, or immediate use in an assay.

2. The method according to claim 1 wherein the lysate of step (a) is obtained in the presence of guanidinium chloride.

3. The method according to any of claims 1 to 2 wherein the purification in step (c) is chromatography.

4. The method according to claim 3 wherein the chromatography is a Ni-IMAC chromatography with said recombinant protein being a His-tagged recombinant protein.

5. The method according to any of claims 1 to 4, comprising the step of desulphonation of the sulphonated version of the recombinant protein.

6. The method according to claim 5, wherein the desulphonation is with a molar excess of reducing agent.

7. The method according to claim 6 wherein the reducing agent is DTT.

8. The method according to any of claims 1 to 7, comprising the step of storage in the presence of a molar excess of DTT, or immediate use in an assay.

## Patentansprüche

1. Verfahren zur Aufreinigung eines Cystein enthaltenden, rekombinant exprimierten HCV-NS3-Proteins, das die Schritte (a) und (c) sowie wenigstens 1 der folgenden Schritte (b), (d) oder (e) umfaßt:
(a) Sulfonierung eines Lysats von rekombinanten Wirtszellen;
(b) Behandlung mit einem zwitterionischen Detergens, vorzugsweise nach Abtrennung der Zelltrümmer;
(c) Aufreinigung der sulfonierten Version des rekombinanten Proteins oder Aufreinigung der sulfonierten Version des rekombinanten Proteins mit anschließender Abtrennung des Detergens;
(d) Desulfonierung des sulfonierten rekombinanten Proteins;
(e) Lagerung in Gegenwart eines molaren Überschusses an DTT oder sofortige Verwendung in einem Test.

2. Verfahren nach Anspruch 1, wobei das Lysat in Schritt (a) in Gegenwart von Guanidiniumchlorid gewonnen wird.

3. Verfahren nach einem der Ansprüche 1 bis 2, wobei es sich bei der Aufreinigung in Schritt (c) um eine Chromatographie handelt.

4. Verfahren nach Anspruch 3, wobei es sich bei der Chromatographie um eine Ni-IMAC-Chromatographie und bei dem rekombinanten Protein um ein rekombinantes Protein mit einem His-Tag handelt.

5. Verfahren nach einem der Ansprüche 1 bis 4, das den Schritt der Desulfonierung der sulfonierten Version des rekombinanten Proteins umfaßt.

6. Verfahren nach Anspruch 5, wobei die Desulfonierung in Gegenwart eines molaren Überschusses an Reduktionsmittel erfolgt.

7. Verfahren nach Anspruch 6, wobei es sich bei dem Reduktionsmittel um DTT handelt.

8. Verfahren nach einem der Ansprüche 1 bis 7, das den Schritt der Lagerung in Gegenwart eines molaren Überschusses an DTT oder der sofortigen Verwendung in einem Test umfaßt.

## Revendications

1. Procédé pour purifier une protéine NS3 de HCV, exprimée de manière recombinante et contenant de la cystéine, comprenant les étapes (a) et (c) et au moins l'une des étapes (b), (d) ou (e) suivantes :
(a) sulfonation d'un lysat provenant de cellules hôtes recombinantes,
(b) traitement avec un détergent zwitterionique, de préférence après un retrait des débris cellulaires,
(c) purification de la version sulfonée de la protéine recombinante ou purification de la version sulfonée de la protéine recombinante avec un retrait ultérieur du détergent,
(d) désulfonation de la protéine recombinante sulfonée,
(e) stockage en présence d'un excès molaire de DTT ou utilisation immédiate dans une analyse.

2. Procédé selon la revendication 1, dans lequel le lysat de l'étape (a) est obtenu en présence de chlorure de guanidinium.

3. Procédé selon l'une quelconque des revendications 1 à 2, dans lequel la purification de l'étape (c) est une chromatographie.

4. Procédé selon la revendication 3, dans lequel la chromatographie est une chromatographie IMAC à Ni, avec ladite protéine recombinante étant une protéine recombinante étiquetée par His.

5. Procédé, selon l'une quelconque des revendications 1 à 4, comprenant l'étape de désulfonation de la version sulfonée de la protéine recombinante.

6. Procédé selon la revendication 5, dans lequel la désulfonation se fait avec un excès molaire d'un agent réducteur.

7. Procédé selon la revendication 6, dans lequel l'agent réducteur est le DTT.

8. Procédé selon l'une quelconque des revendications 1 à 7, comprenant l'étape de stockage en présence d'un excès molaire de DTT ou une utilisation immédiate dans une analyse.
